# EUROPÄISCHE PATENTANMELDUNG

(11) **EP 1 170 021 A2**
(43) Veröffentlichungstag der Anmeldung: **09.01.2002**
(21) Anmeldenummer: 01250164.9
(22) Anmeldetag: 14.05.2001
(51) Int. Cl.: A61K 49/00

(54) **Konjugate von Peptiden und Lanthanid-Chelaten für die Fluoreszenzdiagnostik**

(30) Priorität: 15.05.2000 US 571407
(71) Anmelder: Shering Aktiengesellschaft, 13353 Berlin (DE)
(72) Erfinder: Bauer, Michael, Dr., 13503 Berlin (DE); Becker, Andreas, Dr., 85570 Markt Schwaben (DE); Licha, Kai, Dr., 14612 Falkensee (DE); Bornhop, Darryl, Dr., Lubbock, Texas 79413 (US); Platzek, Johannes, Dr., 12621 Berlin (DE)

(57) **Zusammenfassung**

Die Erfindung betrifft neue Verbindungen zur Fluoreszenzdiagnostik, die Verwendung dieser Verbindungen sowie ein Verfahren zu deren Herstellung.

## Beschreibung

Die Erfindung liegt auf dem Gebiet der Fluoreszenzdiagnostik und betrifft neue Verbindungen, welche in den Patentansprüchen definiert sind. Die Erfindung betrifft weiterhin die Verwendung dieser Verbindungen sowie ein Verfahren zu deren Herstellung.

Die Verwendung von Lanthanid-Chelaten in der endoskopischen Diagnostik wurde bereits beschrieben (Houlne et al., Journal of Biomedical Optics, April 1998, Vol. 3, No. 2, Seite 145 ff.; WO 97/40055). Insbesondere Terbium- und Europiumkomplexe mit Tri- und Tetraazamakrocyclischen Chelatoren wurden bereits erfolgreich als fluoreszierende in vitro- oder in vivo-Diagnostika eingesetzt. Werden die Verbindungen mit Licht geeigneter Wellenlänge bestrahlt, senden sie eine langlebige Fluoreszenz im sichtbaren Bereich aus. Dies gilt auch für die in WO 99/46600 beschriebenen Europiumkomplexe.

Für eine medizinische Anwendung ist es jedoch nicht nur notwendig, daß ein Fluoreszenzdiagnostikum Licht bestimmter Wellenlänge absorbiert und Licht anderer Wellenlänge emittiert. Vielmehr ist die Gewebeselektivität der Substanz eine Grundvoraussetzung dafür, daß der behandelnde Arzt eine sichere Diagnose stellen kann. Dies ist insbesondere bei der Tumorerkennung eine wichtige Anforderung an ein Diagnostikum, um falschpositive Resultate zu vermeiden. Die bekannten Verbindungen des Standes der Technik weisen noch keine ausreichende Gewebeselektivität auf. Verbesserungen betreffend die selektive Anreicherung einer fluoreszierenden Substanz in erkranktem Gewebe sind daher wünschenswert.

Aufgabe der Erfindung ist es daher, Verbindungen bereitzustellen, die sich selektiv in erkranktem Gewebe anreichern und nach Anregung mit Licht bestimmter Wellenlänge eine langlebige Fluoreszenz aussenden.

Diese Aufgabe wird dadurch gelöst, daß Konjugate aus Komplexverbindungen und rezeptorbindenden Peptiden bereitgestellt werden. Die rezeptorbindenden Peptide reichern sich selektiv in erkranktem Gewebe an.

Die neuen Verbindungen sind Verbindungen der allgemeinen Formel (I)

A¹ - L¹ - (X)ₘ - L² - A² (I)

worin
- X: für eine beliebige α, β oder γ-Aminosäure mit D- oder L-Konfiguration und
- m: für eine Zahl von 5 bis 30 steht,
wobei die resultierende Aminosäuresequenz (X)ₘ, welche aus beliebigen aneinandergereihten Aminosäuren X besteht, geradkettiger Natur oder über eine Disulfidbrücke zwischen zwei Cysteinen oder Homocysteinen oder amidisch zwischen N- und C-Terminus cyclisiert sein kann und für die Aminosäuresequenz des vasoaktiven intestinalen Peptids (VIP), des Somatostatins oder des Neurotensins, oder für Fragmente, Teilsequenzen, Derivate oder Analoga des VIP, des Somatostatins oder des Neurotensins steht,
- A¹: für ein Wasserstoffatom, eine offenkettige oder cyclische Polyaminopolycarbonsäure oder Polyaminopolyphosphonsäure steht, welche eine Arylgruppe oder einen Heteroaromaten enthält und ein Metallatom der Ordnungszahlen 57 bis 83 komplexiert,
- L¹ und L²: unabhängig voneinander einen Acetylrest oder einen Alkylrest mit bis zu 10 C-Atomen, der gegebenenfalls mit 1 bis 3 Carboxygruppen und/oder 1 bis 6 Hydroxygruppen und/oder 1 bis 6 Amidgruppen substituiert sein kann, oder einen Poly(oxyethylen)rest mit 2 bis 30 -CH₂CH₂O-Einheiten darstellen,
- A²: für eine Hydroxygruppe, eine Aminogruppe, eine offenkettige oder cyclische Polyaminopolycarbonsäure oder Polyaminopolyphosphonsäure steht, welche eine Arylgruppe oder einen Heteroaromaten enthält und ein Metallatom der Ordnungszahlen 57 bis 83 komplexiert,
unter der Bedingung, daß mindestens einer der Reste A¹ oder A² eine offenkettige oder cyclische Polyaminopolycarbonsäure oder Polyaminopolyphosphonsäure darstellt, welche eine Arylgruppe oder einen Heteroaromaten enthält und ein Metallatom der Ordnungszahlen 57 bis 83 komplexiert,
wobei für den Fall, daß A¹ und/oder A² eine offenkettige oder cyclische Polyaminopolycarbonsäure oder Polyaminopolyphosphonsäure darstellen, welche eine Arylgruppe oder einen Heteroaromaten enthält und ein Metallatom der Ordnungszahlen 57 bis 83 komplexiert, A¹ an die N-terminale Aminogruppe und A² an eine Aminogruppe der Aminosäure Lysin oder an eine Hydroxygruppe der Aminosäure Serin oder an die Mercaptogruppe der Aminosäure Cystein oder Homocystein in beliebiger Position innerhalb der Aminosäuresequenz (X)ₘ geknüpft ist,
und deren physiologisch verträgliche Salze.

Der Begriff "Aminosäure" im Sinne dieser Erfindung steht für eine Carbonsäure mit einer oder mehreren Aminogruppen im Molekül sowie für cyclisierte Aminosäuren wie z.B. Pyroglutaminsäure.

Bevorzugte Chelatoren (Komplexbildner) sind Derivate der Diethylentriaminpentaessigsäure (DTPA) und des 1,4,7,10-Tetraazacyclododecans (DOTA).

Von den DTPA-Derivaten sind solche der allgemeinen Formeln (II), (III) und (IV) bevorzugt: 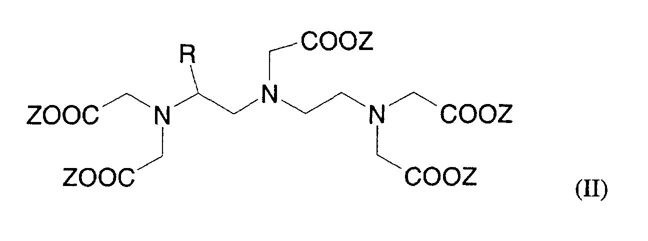 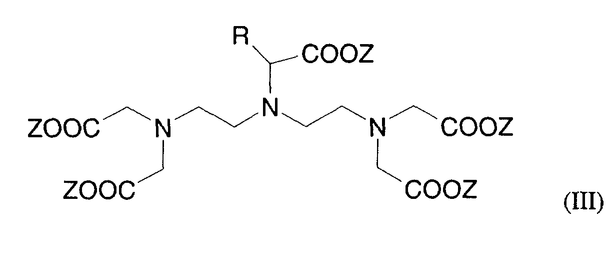 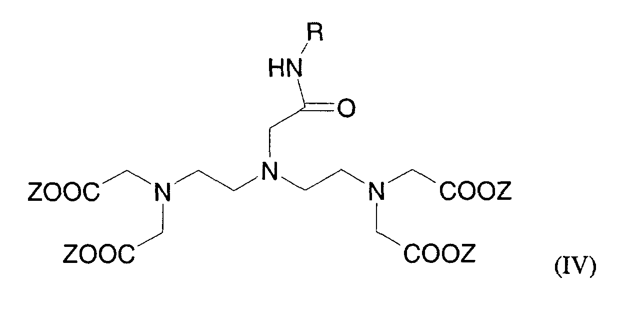 worin Z unabhängig voneinander für ein Wasserstoffatom oder ein Metallionenäquivalent eines Elementes der Ordnungszahlen 57 bis 83 steht, und worin R für einen offenkettigen oder cyclischen, verzweigten oder unverzweigten C₁-C₁₀-Alkylrest steht, welcher mindestens einen aromatischen Ring sowie gegebenenfalls 1 bis 5 Sauerstoffatome, 1 bis 3 Carboxygruppen und/oder 1 bis 3 Amidgruppen enthält, oder worin R einen der folgenden Reste darstellt: 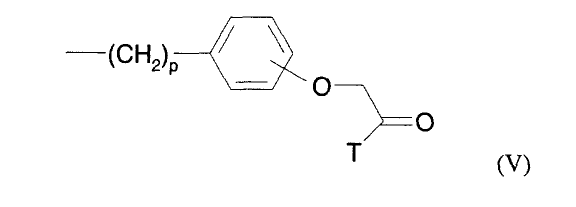 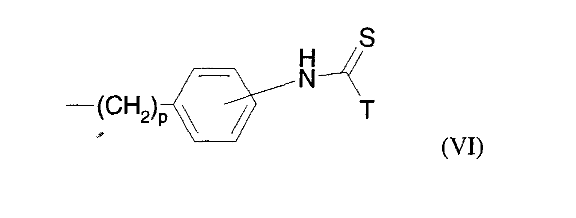 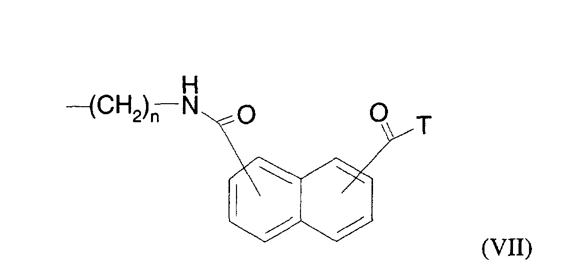 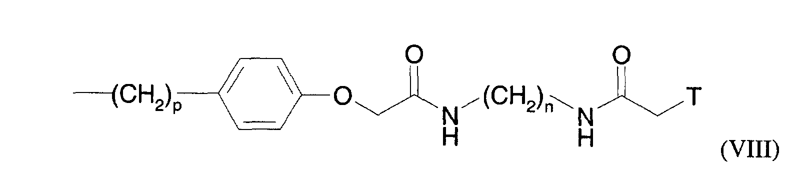 worin T jeweils die Verknüpfungsstelle mit dem Peptid darstellt, p für eine Zahl 0 oder 1 steht und n für eine Zahl zwischen 2 und 6 steht.

Von den DOTA-Derivaten sind solche der allgemeinen Formel (IX) bevorzugt: 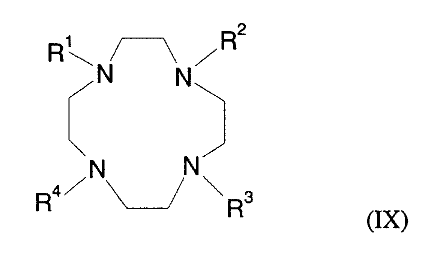 worin R¹ einen Rest ―CHR⁵-COM darstellt, worin M für eine OZ-Gruppe steht, mit Z in der obengenannten Bedeutung, oder die Verknüpfung zum Peptid darstellt, und worin R⁵ für einen Rest (VIII) oder
für eine C₁-C₂₀-Alkylgruppe steht, welche mindestens eine Arylgruppe oder einen Heteroaromaten, welche gegebenenfalls mit einem Halogenatom substituiert sein können, und mindestens eine weitere COOH-Gruppe oder eine Isothiocyanatgruppe enthält, und welche gegebenenfalls 1 bis 3 Sauerstoffatome und/oder 1 bis 3 Amidgruppen enthält,
und worin R² bis R⁴ unabhängig voneinander einen Rest CH₂COOZ, einen Phosphonsäurerest oder eine Gruppe ―(CH₂)ₚ-Y darstellen, in der p für 0 oder 1 steht und Y einen gegebenenfalls substituierten Heteroaromaten darstellt.

Weitere bevorzugte Komplexbildner sind die folgenden Verbindungen der allgemeinen Formel (X): 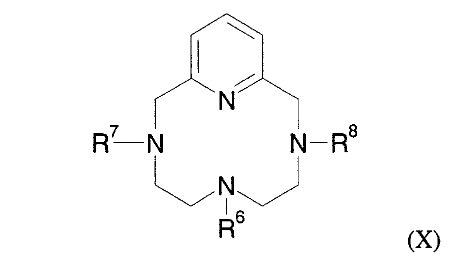 worin R⁶ einen Rest―CHR⁹-COM darstellt, worin M für eine OZ-Gruppe steht oder die Verknüpfung zum Peptid darstellt, und worin R⁹ für einen Rest (VIII) oder eine C₁-C₆-Alkylgruppe steht, welche gegebenenfalls eine weitere COOH-Gruppe oder eine Isothiocyanatgruppe enthält, und welche gegebenenfalls 1 bis 2 Sauerstoffatome und/oder 1 bis 2 Amidgruppen enthält,
und worin R⁷ und R⁸ unabhängig voneinander einen Rest CH₂COOZ oder einen Phosphonsäurerest darstellen.

Die Metallkomplexe enthalten bevorzugt ein Terbium- oder Europiumkation.

Die rezeptorbindenden Peptide sind das native vasoaktive intestinale Peptid (VIP), das Somatostatin oder das Neurotensin sowie Fragmente, Teilsequenzen, Derivate oder Analoga des VIP, des Somatostatins oder des Neurotensins.

Das native VIP wird durch die Aminosäuresequenz HSDAVFTDNYTRLRKQMAVKKYLNSILN beschrieben.

Das Somatostatin wird durch die Aminosäuresequenz 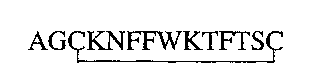
AGCKNFFWKTFTSC
 beschrieben.

Das Neurotensin wird durch die Aminosäuresequenz Pyroglutaminsäure-LYENKPRRPYIL beschrieben.

Als Fragmente, Teilsequenzen, Derivate oder Analoga des VIP seien beispielhaft die folgenden Verbindungen genannt:

RLRKQMAVKKYLNSILN RLRKQMAVKKYLNSIL RLRKQMAVKKYLNSI
LRKQMAVKKYLNSILN LRKQMAVKKYLNSIL LRKQMAVKKYLNSI
RKQMAVKKYLNSILN RKQMAVKKYLNSIL RKQMAVKKYLNSI
KQMAVKKYLNSILN KQMAVKKYLNSIL KQMAVKKYLNSI
QMAVKKYLNSILN QMAVKKYLNSIL QMAVKKYLNSI
MAVKKYLNSILN MAVKKYLNSIL MAVKKYLNSI
AVKKYLNSILN AVKKYLNSIL AVKKYLNSI

RLRKQMAVKKYLNS | RLRKQMAVKKYLN | RLRKQMAVKKYL
LRKQMAVKKYLNS | LRKQMAVKKYLN | LRKQMAVKKYL
RKQMAVKKYLNS | RKQMAVKKYLN | RKQMAVKKYL
KQMAVKKYLNS | KQMAVKKYLN | KQMAVKKYL
QMAVKKYLNS | QMAVKKYLN | QMAVKKYL
MAVKKYLNS | MAVKKYLN | MAVKKYL
AVKKYLNS | AVKKYLN | AVKKYL

Weitere Beispiele für Fragmente, Teilsequenzen, Derivate oder Analoga des VIP sind die folgenden Verbindungen:

rlrkqmavkkylnsiln | rlrkqmavkkylnsil | rlrkqmavkkylnsi
lrkqmavkkylnsiln | lrkqmavkkylnsil | lrkqmavkkylnsi
rkqmavkkylnsiln | rkqmavkkylnsil | rkqmavkkylnsi
kqmavkkylnsiln | kqmavkkylnsil | kqmavkkylnsi
qmavkkylnsiln | qmavkkylnsil | qmavkkylnsi
mavkkylnsiln | mavkkylnsil | mavkkylnsi

avkkylnsiln avkkylnsil avkkylnsi

RLRKQMAvKKyLNSILN RLRKQMAvKKyLNSIL RLRKQMAvKKyLNSI
LRKQMAvKKyLNSILN LRKQMAvKKyLNSIL LRKQMAvKKyLNSI
RKQMAvKKyLNSILN RKQMAvKKyLNSIL RKQMAvKKyLNSI
KQMAvKKyLNSILN KQMAvKKyLNSIL KQMAvKKyLNSI
QMAvKKyLNSILN QMAvKKyLNSIL QMAvKKyLNSI
MAvKKyLNSILN MAvKKyLNSIL MAvKKyLNSI
AvKKyLNSILN AvKKyLNSIL AvKKyLNSI

Weiter können 1 bis m Aminosäuren unabhängig voneinander gegen ihre jeweilige D-Aminosäure oder gegen andere L- oder D-Aminosäuren ausgetauscht sein, wobei m die oben angegebene Bedeutung hat. Sämtliche Aminosäuren (X)ₘ können auch gegen ihre jeweilige D-Aminosäure ausgetauscht sein. Als Fragmente, Teilsequenzen, Derivate oder Analoga des vasoaktiven intestinalen Peptides können auch retrosynthetische Aminosäuresequenzen ausgewählt sein. Bei diesen retrosynthetischen Aminosäuresequenzen können 1 bis m Aminosäuren gegen die jeweilige D-Aminosäure ausgetauscht sein.

Ferner seien im folgenden weitere Beispiele für VIP-Analoga aufgeführt:

FSDAVFTDNY TRLRKQMAVK KYLNSILN
ISDAVFTDNY TRLRKQMAVK KYLNSILN
LSDAVFTDNY TRLRKQMAVK KYLNSILN
HFDAVFTDNY TRLRKQMAVK KYLNSILN
HHDAVFTDNY TRLRKQMAVK KYLNSILN
HIDAVFTDNY TRLRKQMAVK KYLNSILN
HLDAVFTDNY TRLRKQMAVK KYLNSILN
HMDAVFTDNY TRLRKQMAVK KYLNSILN
HQDAVFTDNY TRLRKQMAVK KYLNSILN
HTDAVFTDNY TRLRKQMAVK KYLNSILN

HVDAVFTDNY TRLRKQMAVK KYLNSILN
HWDAVFTDNY TRLRKQMAVK KYLNSILN
HYDAVFTDNY TRLRKQMAVK KYLNSILN
HSAAVFTDNY TRLRKQMAVK KYLNSILN
HSEAVFTDNY TRLRKQMAVK KYLNSILN
HSFAVFTDNY TRLRKQMAVK KYLNSILN
HSHAVFTDNY TRLRKQMAVK KYLNSILN
HSIAVFTDNY TRLRKQMAVK KYLNSILN
HSLAVFTDNY TRLRKQMAVK KYLNSILN
HSMAVFTDNY TRLRKQMAVK KYLNSILN
HSWAVFTDNY TRLRKQMAVK KYLNSILN
HSDFVFTDNY TRLRKQMAVK KYLNSILN
HSDGVFTDNY TRLRKQMAVK KYLNSILN
HSDMVFTDNY TRLRKQMAVK KYLNSILN
HSDQVFTDNY TRLRKQMAVK KYLNSILN
HSDSVFTDNY TRLRKQMAVK KYLNSILN
HSDWVFTDNY TRLRKQMAVK KYLNSILN
HSDYVFTDNY TRLRKQMAVK KYLNSILN
HSDAFFTDNY TRLRKQMAVK KYLNSILN
HSDAIFTDNY TRLRKQMAVK KYLNSILN
HSDALFTDNY TRLRKQMAVK KYLNSILN
HSDAMFTDNY TRLRKQMAVK KYLNSILN
HSDATFTDNY TRLRKQMAVK KYLNSILN
HSDAWFTDNY TRLRKQMAVK KYLNSILN
HSDAYFTDNY TRLRKQMAVK KYLNSILN
HSDAVKTDNY TRLRKQMAVK KYLNSILN
HSDAVFVDNY TRLRKQMAVK KYLNSILN
HSDAVFWDNY TRLRKQMAVK KYLNSILN
HSDAVFTDNW TRLRKQMAVK KYLNSILN
HSDAVFTDNY TRRRKQMAVK KYLNSILN
HSDAVFTDNY TRWRKQMAVK KYLNSILN

HSDAVFTDNY TRLRFQMAVK KYLNSILN
HSDAVFTDNY TRLRLQMAVK KYLNSILN
HSDAVFTDNY TRLRMQMAVK KYLNSILN
HSDAVFTDNY TRLRRQMAVK KYLNSILN
HSDAVFTDNY TRLRKAMAVK KYLNSILN
HSDAVFTDNY TRLRKFMAVK KYLNSILN
HSDAVFTDNY TRLRKIMAVK KYLNSILN
HSDAVFTDNY TRLRKKMAVK KYLNSILN
HSDAVFTDNY TRLRKLMAVK KYLNSILN
HSDAVFTDNY TRLRKMMAVK KYLNSILN
HSDAVFTDNY TRLRKRMAVK KYLNSILN
HSDAVFTDNY TRLRKVMAVK KYLNSILN
HSDAVFTDNY TRLRKWMAVK KYLNSILN
HSDAVFTDNY TRLRKYMAVK KYLNSILN
HSDAVFTDNY TRLRKQFAVK KYLNSILN
HSDAVFTDNY TRLRKQIAVK KYLNSILN
HSDAVFTDNY TRLRKQKAVK KYLNSILN
HSDAVFTDNY TRLRKQLAVK KYLNSILN
HSDAVFTDNY TRLRKQQAVK KYLNSILN
HSDAVFTDNY TRLRKQRAVK KYLNSILN
HSDAVFTDNY TRLRKQWAVK KYLNSILN
HSDAVFTDNY TRLRKQMFVK KYLNSILN
HSDAVFTDNY TRLRKQMIVK KYLNSILN
HSDAVFTDNY TRLRKQMKVK KYLNSILN
HSDAVFTDNY TRLRKQMLVK KYLNSILN
HSDAVFTDNY TRLRKQMMVK KYLNSILN
HSDAVFTDNY TRLRKQMQVK KYLNSILN
HSDAVFTDNY TRLRKQMRVK KYLNSILN
HSDAVFTDNY TRLRKQMVVK KYLNSILN
HSDAVFTDNY TRLRKQMWVK KYLNSILN
HSDAVFTDNY TRLRKQMYVK KYLNSILN

HSDAVFTDNY TRLRKQMAAK KYLNSILN
HSDAVFTDNY TRLRKQMAIK KYLNSILN
HSDAVFTDNY TRLRKQMALK KYLNSILN
HSDAVFTDNY TRLRKQMAVR KYLNSILN
HSDAVFTDNY TRLRKQMAVK RYLNSILN
HSDAVFTDNY TRLRKQMAVK WYLNSILN
HSDAVFTDNY TRLRKQMAVK KFLNSILN
HSDAVFTDNY TRLRKQMAVK KWLNSILN
HSDAVFTDNY TRLRKQMAVK KYLASILN
HSDAVFTDNY TRLRKQMAVK KYLFSILN
HSDAVFTDNY TRLRKQMAVK KYLISILN
HSDAVFTDNY TRLRKQMAVK KYLMSILN
HSDAVFTDNY TRLRKQMAVK KYLSSILN
HSDAVFTDNY TRLRKQMAVK KYLVSILN
HSDAVFTDNY TRLRKQMAVK KYLWSILN
HSDAVFTDNY TRLRKQMAVK KYLNNILN
HSDAVFTDNY TRLRKQMAVK KYLNRILN
HSDAVFTDNY TRLRKQMAVK KYLNWILN
HSDAVFTDNY TRLRKQMAVK KYLNYILN
HSDAVFTDNY TRLRKQMAVK KYLNSLLN
HSDAVFTDNY TRLRKQMAVK KYLNSSLN
HSDAVFTDNY TRLRKQMAVK KYLNSWLN
HSDAVFTDNY TRLRKQMAVK KYLNSYLN
HSDAVFTDNY TRLRKQMAVK KYLNSIFN
HSDAVFTDNY TRLRKQMAVK KYLNSIIN
HSDAVFTDNY TRLRKQMAVK KYLNSIWN
HSDAVFTDNY TRLRKQMAVK KYLNSILW

Weiter können VIP-Analoga verwendet werden, die durch die folgende Formel beschrieben werden: HSDAVFTX¹X²Y X³RLRKQMAVK KYLNSILN, worin X¹, X² und X³ jede beliebige Aminosäure darstellen können.

Als Fragmente, Teilsequenzen, Derivate oder Analoga des Somatostatins können folgende Sequenzen ausgewählt sein:

AGCKNFFwKTFTSC    AGcKNFFwKTFTSC
AGCKNFFwKTFTSc    AGcKNFFwKTFTSc
CKNFFwKTFTSC    cKNFFwKTFTSC
fFYwKVFT
fCFwKVCT    fcFwKVCT
fCYwKVCT    fcYwKVCT
fCFwKTCT    fcFwKTCT
fCYwKTCT    fcYwKTCT
D-NaI-CYwKVC    D-NaI-cYwKVC
fCywK-Abu-C-NaI    fcywK-Abu-C-NaI

Als Fragmente, Teilsequenzen, Derivate oder Analoga des Neurotensins können folgende Sequenzen ausgewählt sein:

pGlu-LYQNKPRRPFIL | pGlu-LYENKPRRPYI
pGlu-LYENKPRRPyIL | pGlu-LYQNKPRRPfIL | pGlu-LYENKPRRPY
pGlu-LYQNKPRRPYIL | pGlu-LYENKPRRPWIL | pGlu-LYENKPRRP
pGlu-LYQNKPRRPyIL | pGlu-LYENKPRRPwIL | pGlu-LYENKPRR
pGlu-LYENKPRRPFIL | pGlu-LYQNKPRRPWIL | pGlu-LYENKPR
pGlu-LYENKPRRPfIL | pGlu-LYQNKPRRPwIL | pGlu-LYENKP

NKPRRPYIL    NKPRRPyIL    NKPRRPfIL    NKPRRPwIL
KPRRPYIL    KPRRPyIL    KPRRPfIL    KPRRPwIL
PRRPYIL    PRRPyIL    PRRPfIL    PRRPwIL
RRPYIL    RRPyIL    RRPfIL    RRPwIL

Die Terbiumkomplexe emittieren nach Einstrahlung von nicht sichtbarem Licht der Wellenlänge 250 bis 450 nm eine langlebige Fluoreszenz im Millisekundenbereich, welche Wellenlängen im Bereich von 480 bis 600 nm aufweist. In diesem Wellenlängenbereich ist das menschliche Auge am empfindlichsten. Die langlebige Fluoreszenz der erfindungsgemäßen Verbindungen überdauert die bei der endoskopischen Untersuchung auftretende Autofluoreszenz des Gewebes. Die endoskopische Diagnostik von oberflächlichen Tumoren wird durch die erfindungsgemäßen Verbindungen wesentlich erleichtert. Ähnliche Vorteile ergeben sich durch die Möglichkeit der topischen Applikation (z.B. durch Versprühen).

Eine Anreicherung im krankhaften Gewebe wird auch durch i.v.-Applikation erreicht. Werden die erfindungsgemäßen Substanzen während einer Operation interstitiell appliziert, reichern sie sich in den sogenannten "Wächter-Lymphknoten" an. Der Chirurg kann dadurch diesen Lymphknoten durch seine Fluoreszenz besser erkennen und entsprechende therapierelevante Entscheidungen treffen.

Die erfindungsgemäßen Substanzen sind daher besonders geeignet zur In-vivo-Diagnostik von Tumoren, anderen erkrankten Gewebebereichen oder Adenomen mittels optischer Detektionsverfahren, oder zur In-vivo-Fluoreszenzdiagnostik von Tumoren, Tumorzellen und/oder entzündlichen Geweben mittels endoskopischer Verfahren im Gastrointestinaltrakt, Oesophagus, Bronchialtrakt, der Blase oder der Zervix.

Ein weiterer Gegenstand der Erfindung ist ein Verfahren zur endoskopischen In-vivo-Fluoreszenzdiagnostik unter Verwendung der erfindungsgemäßen Verbindungen, wobei dem Patienten die Verbindungen topisch durch Versprühen im Gastrointestinaltrakt, Oesophagus, der Blase, oder durch Inhalation den Bronchien zugeführt werden. Im Falle des Versprühens im Gastrointestinaltrakt, im Oesophagus und der Blase wird der nicht gebundene, überschüssige Anteil der Verbindung anschließend durch Waschen entfernt. Schließlich wird die endoskopische Untersuchung durch örtliche Anregung mit einer aus dem Spektralbereich von 250 bis 450 nm ausgewählten Anregungswellenlänge und durch ortsabhängige Detektion der spezifischen, von der Verbindung emittierten Fluoreszenzstrahlung durchgeführt.

Die Synthese der Verbindungen erfolgt nach den dem Fachmann bekannten Verfahren. Detaillierte Synthesevorschriften befinden sich in den nachfolgenden Beispielen. Eine besonders vorteilhafte Möglichkeit der Synthese der Konjugate ergibt sich durch den Einbau einer Essigsäureeinheit an den Aromaten des Metallkomplexes. Diese Carbonsäure, die sich außerhalb des Metallkomplexes befindet, ist besonders gut aktivierbar, wobei die Stabilität des Komplexes durch die Aktivierung nicht beeinflußt wird. Dadurch kann der Metallkomplex unter besonders milden Reaktionsbedingungen an ein Peptid gekoppelt werden. Der Vorteil nur einer aktivierbaren Gruppe, wie z.B. einer Carboxylgruppe, oder einer bereits aktivierten Gruppe, wie z.B. einem Isothiocyanat, einer Halogenalkylgruppe oder einer Halogenacetylgruppe, besteht darin, daß eine chemisch einheitliche Kopplung erfolgen kann. Die Halogenacetylgruppe hat den besonderen Vorteil, daß eine chemisch einheitliche Kopplung an die Mercaptogruppe des Cysteins oder Homocysteins erfolgt. Diese Kopplung kann in Lösung an das ungebundene und von Schutzgruppen befreite Peptid erfolgen. Durch die aktivierten Gruppen ist eine Kopplung an Peptide möglich, ohne daß Nebenreaktionen auftreten. Bei dem neuen Herstellungsverfahren wird demnach zunächst ein Metallkomplex hergestellt, welcher anschließend durch Aminolyse des entsprechenden Aktivesters an ein Peptid gekoppelt wird.

Gegenstand der Erfindung ist auch ein optisches Diagnostikum zur in-vivo-Diagnostik erkrankter Gewebebereiche, welches mindestens eine erfindungsgemäße Verbindung zusammen mit den üblichen Hilfs- und/oder Trägerstoffen sowie Verdünnungsmitteln enthält. Derartige galenische Zubereitungen werden vorteilhafterweise durch Sterilfiltration der entsprechenden Lösungen hergestellt.

Erfindungsgemäße Verbindungen, die paramagnetische Metallatome enthalten, sind darüber hinaus für die Magnetresonanz-Bildgebung und ―Spektroskopie geeignet.

Die nachfolgenden Beispiele erläutern die Erfindung.

### Beispiel 1

N-Terminal verknüpftes Peptid-Konjugat mit dem Terbiumkomplex des Dinatriumsalzes von N,N-Bis-{2-[N',N'-bis-(carboxymethyl)-amino]-ethyl}-L-3-[(4-carboxymethoxy)-phenyl]-alanin

### a) N,N-Bis-{2-[N',N'-bis-(benzyloxycarbonyl-methyl)-amino]-ethyl}-L-3-[(4-benzyloxycarbonylmethoxy)-phenyl]-alaninbenzylester

1,9 g (2 mmol) N,N-Bis-{2-[N',N'-bis-(benzyloxycarbonyl-methyl)-amino]-ethyl}-L-tyrosinbenzylester (WO 96/26180, Beispiel 1a) werden in 10 ml wasserfreiem N,N-Dimethylformamid gelöst und bei 0°C unter Argon mit 53 mg (2,2 mmol) Natriumhydriddispersion (60 % in Mineralöl) versetzt. Man läßt den Ansatz 15 Minuten rühren, gibt dann 0,5 g (2,3 mmol) Bromessigsäurebenzylester zu, läßt die Reaktionsmischung auf Raumtemperatur kommen und rührt weitere sechs Stunden. Zur Aufarbeitung wird der Ansatz in Toluol aufgenommen und mehrmals gegen wässrige Natriumhydrogencarbonatlösung ausgeschüttelt. Die organische Phase wird abgetrennt, über Magnesiumsulfat getrocknet, filtriert und eingedampft. Der ölige Rückstand wird an Kieselgel chromatographiert, die produkthaltigen Fraktionen werden vereint und eingedampft.
Ausbeute: 2,0 g (91 % d. Th.) farbloses Öl.

| Analyse (bezogen auf lösungsmittelfreie Substanz): | | | | |
|---|---|---|---|---|
| ber. | C 71,09 | H 6,15 | N 3,83 | O 18,94 |
| gef. | C 71,01 | H 6,28 | N 3,67 | |

### b) N,N-Bis-{2-[N',N'-bis-(carboxymethyl)-amino]-ethyl}-L-3-[(4-carboxymethoxy)-phenyl]-alanin

Eine Lösung von 1,9 g (1,7 mmol) N,N-Bis-{2-[N',N'-bis-(benzyloxycarbonylmethyl)-amino]-ethyl}-L-3-[(4-benzyloxycarbonylmethoxy)-phenyl]-alaninbenzylester (Beispiel 1a) in 15 ml Methanol wird mit 0,2 g Palladium auf Aktivkohle (10 % Pd) versetzt und unter Wasserstoffatmosphäre über Nacht kräftig gerührt. Anschließend wird filtriert und das Filtrat im Vakuum eingedampft.
Ausbeute: 0,9 g (95 % d. Th.) farbloses Öl.

| Analyse (bezogen auf lösungsmittelfreie Substanz): | | | | |
|---|---|---|---|---|
| ber. | C 49,55 | H 5,60 | N 7,54 | O 37,31 |
| gef. | C 49,37 | H 5,72 | N 7,40 | |

### c) Terbiumkomplex des Dinatriumsalzes von N,N-Bis-{2-[N',N'-bis-(carboxymethyl)-amino]-ethyl}-L-3-[(4-carboxymethoxy)-phenyl]-alanin

0,8 g (1,4 mmol) N,N-Bis-{2-[N',N'-bis-(carboxymethyl)-amino]-ethyl}-L-3-[(4-carboxymethoxy)-phenyl]-alanin (Beispiel 1b) werden zusammen mit 0,7 g (1,4 mmol) Terbiumcarbonat in 10 ml Wasser vier Stunden bei 50°C gerührt. Anschließend wird filtriert, eingedampft und an Kieselgel RP-18 chromatographisch (Wasser/Acetonitril) gereinigt.
Ausbeute: 1,0 g (94 % der Theorie) farbloser Feststoff.

| Analyse (bezogen auf lösungsmittelfreie Substanz): | | | | | | |
|---|---|---|---|---|---|---|
| ber. | C 36,48 | H 3,46 | N 5,55 | Na 6,07 | Tb 20,98 | O 27,46 |
| gef. | C 36,24 | H 3,55 | N 5,39 | Na 5,93 | Tb 20,84 | |

### d) N-Terminal verknüpftes Peptid-Konjugat mit dem Terbiumkomplex des Dinatriumsalzes von N,N-Bis-{2-[N',N'-bis-(carboxymethyl)-amino]-ethyl}-L-3- [(4-carboxymethoxy)-phenyl]-alanin

0,3 g (0,4 mmol) Terbiumkomplex des Dinatriumsalzes von N,N-Bis-{2-[N',N'-bis-(carboxymethyl)-amino]-ethyl}-L-3- [(4-carboxymethoxy)-phenyl]-alanin (Beispiel 1c) werden in 2 ml Dimethylsulfoxid gelöst und bei 60°C mit 50 mg (0,45 mmol) N-Hydroxysuccinimid und 93 mg (0,45 mmol) Dicyclohexylcarbodiimid umgesetzt. Nach einer Stunde kühlt man auf Raumtemperatur ab und setzt mit 35 mg (0,03 mmol) dPhe-cyclo-[Cys-Phe-dTrp-Z-Lys-Thr-Cys]-Thr-OH um. Nach 4 Stunden fällt man das Produkt mit Ether, saugt die Fällung ab und wäscht den Feststoff mehrmals mit Ether. Das Zwischenprodukt wird in Wasser/Methanol aufgenommen und an Palladium auf Aktivkohle hydriert. Nach drei Stunden bei Raumtemperatur wird filtriert und das Filtrat gefriergetrocknet. Das Terbiumkomplex-Peptid-Konjugat kann zur Reinigung dialysiert werden oder per HPLC chromatographiert werden.
Ausbeute: 36 mg (68 % der Theorie).

| Analyse (bezogen auf lösungsmittelfreie Substanz): | | | | | | | |
|---|---|---|---|---|---|---|---|
| ber. | C 48,79 | H 5,00 | N 10,27 | Na 2,59 | Tb 8,97 | S 3,62 | O 20,76 |
| gef. | C 48,62 | H 4,88 | N 9,98 | Na 2,31 | Tb 8,82 | S 3,50 | |

### Beispiel 2

Europiumkomplex des Dinatriumsalzes der 3,6,9-Triaza-3,6,9-tris-(carboxymethyl)-4-(4-carboxymethoxybenzyl)-undecandisäure

### a) 3,6,9-Triaza-3,6,9-tris-(tert.-butoxycarbonylmethyl)-4-(4-tert.-butoxycarbonylmethoxybenzyl)-undecandisäure-di-tert.-butylester

1,56 g (2 mmol) N,N-Bis-{2-[N',N'-bis-(tert.-butyloxycarbonyl-methyl)-amino]-ethyl}-L-tyrosintert.-butylester (DOS 3710730) werden in 8 ml wasserfreiem N,N-Dimethylformamid gelöst und bei 0°C unter Argon mit 53 mg (2,2 mmol) Natriumhydriddispersion (60 % in Mineralöl) versetzt. Man läßt den Ansatz 20 Minuten rühren, gibt dann 0,45 g (2,3 mmol) Bromessigsäure-tert.-butylester zu, läßt die Reaktionsmischung auf Raumtemperatur kommen und rührt weitere fünf Stunden. Zur Aufarbeitung wird der Ansatz in Toluol aufgenommen und mehrmals gegen wässrige Natriumhydrogencarbonatlösung ausgeschüttelt. Die organische Phase wird abgetrennt, über Magnesiumsulfat getrocknet, filtriert und eingedampft. Der ölige Rückstand wird an Kieselgel chromatographiert, die produkthaltigen Fraktionen werden vereint und eingedampft.
Ausbeute: 1,6 g (89 % d. Th.) farbloses Öl.

| Analyse (bezogen auf lösungsmittelfreie Substanz): | | | | |
|---|---|---|---|---|
| ber. | C 63,13 | H 8,91 | N 4,70 | O 23,26 |
| gef. | C 62,94 | H 9,03 | N 4,58 | |

### b) 3,6,9-Triaza-3,6,9-tris-(carboxymethyl)-4-(4-carboxymethoxybenzyl)-undecandisäure

1,5 g (1,7 mmol) 3,6,9-Triaza-3,6,9-tris-(tert.-butoxycarbonylmethyl)-4-(4-tert.-butoxycarbonylmethoxybenzyl)-undecandisäure-di-tert.-butylester (Beispiel 2a) werden in 0,8 ml (11 mmol) Trifluoressigsäure gelöst und 12 Stunden bei Raumtemperatur gerührt. Zur Aufarbeitung verdünnt man mit Wasser und dampft zur Trockne ein. Dieser Vorgang wird mehrmals wiederholt. Anschließend wird eine wäßrige Lösung des Produktes über eine Anionenaustauschersäule gereinigt und die produkthaltigen Fraktionen eingedampft.
Ausbeute: 0,8 g (83 % d. Th.) farbloses Öl.

| Analyse (bezogen auf lösungsmittelfreie Substanz): | | | | |
|---|---|---|---|---|
| ber. | C 49,55 | H 5,60 | N 7,54 | O 37,31 |
| gef. | C 49,39 | H 5,72 | N 7,63 | |

### c) Europiumkomplex des Dinatriumsalzes der 3,6,9-Triaza-3,6,9-tris-(carboxymethyl)-4-(4-carboxymethoxybenzyl)-undecandisäure

0,7 g (1,2 mmol) 3,6,9-Triaza-3,6,9-tris-(carboxymethyl)-4-(4-carboxymethoxybenzyl)-undecandisäure (Beispiel 2b) werden in 5 ml Wasser gelöst und mit 290 mg (0,6 mmol) Europiumcarbonat versetzt. Man addiert 0,5 ml Essigsäure und refluxiert über Nacht. Nach beendeter Komplexierung reinigt man das Rohprodukt über eine Ionenaustauschersäule und lyophilisiert die produkthaltigen Fraktionen.
Ausbeute: 0,8 g (94 % der Theorie) farbloses Lyophilisat.

| Analyse (bezogen auf wasserfreie Substanz): | | | | | |
|---|---|---|---|---|---|
| ber. | C 39,10 | H 4,00 | N 5,95 | Eu 21,51 | O 29,44 |
| gef. | C 38,98 | H 4,13 | N 5,76 | Eu 21,43 | |

Die Umsetzung von Beispiel 2c zum Europiumkomplex-Peptid-Konjugat erfolgt analog zu Beispiel 1d.

### Beispiel 3

Terbiumkomplex des Dinatriumsalzes von N,N-Bis-{2-[N',N'-bis-(carboxymethyl)-amino]-ethyl}-N'-(2-carboxynaphthylcarbonyl)-L-lysin

### a) N,N-Bis-{2-[N',N'-bis-(tert.-butyloxycarbonylmethyl)-amino]-ethyl}-N'-(benzyloxycarbonyl)-L-lysin-tert.-butylester

1,2 g (3,3 mmol) H-Lys(Z)-OtBu*HCl (Bachem) und 2,6 g (7,3 mmol) N,N-Bis-[(tert.-butyloxycarbonyl)-methyl]-2-bromethylamin (M. Williams und H. Rapoport, J. Org. Chem. 58, 1151 (1993)) werden in 15 ml Acetonitril vorgelegt und mit 3 ml 2 n Phosphatpufferlösung (pH 8,0) versetzt. Der Ansatz wird bei Raumtemperatur 20 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin chromatographiert. Die produkthaltigen Fraktionen werden im Vakuum eingedampft.
Ausbeute: 2,5 g (86 % d. Th.) farbloses Öl.

| Analyse (bezogen auf lösungsmittelfreie Substanz): | | | | |
|---|---|---|---|---|
| ber. | C 62,85 | H 8,94 | N 6,37 | O 21,84 |
| gef. | C 62,69 | H 9,02 | N 6,44 | |

### b) N,N-Bis-{2-[N',N'-bis-(tert.-butyloxycarbonylmethyl)-amino]-ethyl}-L-lysin-tert.-butylester

2,3 g (2,6 mmol) N,N-Bis-{2-[N',N'-bis-(tert.-butyloxycarbonylmethyl)-amino]-ethyl}-N'-(benzyloxycarbonyl)-L-lysin-tert.-butylester (Beispiel 3a) werden in 20 ml Ethanol gelöst und nach Zugabe von 0,1 g Palladium auf Aktivkohle (10% Palladium) bis zur beendeten Wasserstoffaufnahme hydriert. Anschließend wurde filtriert und das Filtrat vollständig eingedampft.
Ausbeute: 1,9 g (98 % d. Th.) farbloses Öl.

| Analyse (bezogen auf lösungsmittelfreie Substanz): | | | | |
|---|---|---|---|---|
| ber. | C 61,26 | H 9,74 | N 7,52 | O 21,48 |
| gef. | C 61,12 | H 9,65 | N 7,39 | |

### c) N,N-Bis-{2-[N',N'-bis-(tert.-butyloxycarbonylmethyl)-amino]-ethyl}-N'-(2-carboxynaphthylcarbonyl)-L-lysin-tert.-butylester

1,52 g (2 mmol) N,N-Bis-{2-[N',N'-bis-(tert.-butyloxycarbonylmethyl)-amino]-ethyl}-L-lysin-tert.-butylester (Beispiel 3b) werden in 15 ml Dioxan gelöst, mit 0,56 ml (4 mmol) Triethylamin und 420 mg (2 mmol) Naphthalsäureanhydrid versetzt. Man rührt 20 Stunden bei Raumtemperatur, dampft zur Trockne ein und extrahiert den Rückstand mehrmals mit tert.-Butylmethylether. Der Extrakt wird eingeengt und das Rohprodukt an Kieselgel chromatographiert.
Ausbeute: 1,4 g (74 % d. Th.)

| Analyse (bezogen auf lösungsmittelfreie Substanz): | | | | |
|---|---|---|---|---|
| ber. | C 63,67 | H 8,34 | N 5,94 | O 22,05 |
| gef. | C 63,49 | H 8,26 | N 6,08 | |

### d) N,N-Bis-{2-[N',N'-bis-(carboxymethyl)-amino]-ethyl}-N'-(2-carboxynaphthylcarbonyl)-L-lysin

1,3 g (1,4 mmol) N,N-Bis-{2-[N',N'-bis-(tert.-butyloxycarbonylmethyl)-amino]-ethyl}-N'-(2-carboxynaphthylcarbonyl)-L-lysin-tert.-butylester (Beispiel 3c) werden in 0,7 ml (9 mmol) Trifluoressigsäure gelöst und 6 Stunden bei Raumtemperatur gerührt. Zur Aufarbeitung verdünnt man mit Wasser und dampft zur Trockne ein. Dieser Vorgang wird mehrmals wiederholt. Anschließend wird eine wäßrige Lösung des Produktes über eine Anionenaustauschersäule gereinigt und die produkthaltigen Fraktionen eingedampft.
Ausbeute: 0,75 g (81 % d. Th.) farbloser Feststoff.

| Analyse (bezogen auf lösungsmittelfreie Substanz): | | | | |
|---|---|---|---|---|
| ber. | C 54,38 | H 5,78 | N 8,45 | O 31,39 |
| gef. | C 54,21 | H 5,86 | N 8,61 | |

### e) Terbiumkomplex des Dinatriumsalzes von N,N-Bis-{2-[N',N'-bis-(carboxymethyl)-amino]-ethyl -N'-(2-carboxynaphthylcarbonyl)-L-lysin

0,6 g (0,9 mmol) N,N-Bis-{2-[N',N'-bis-(carboxymethyl)-amino]-ethyl}-N'-(2-carboxynaphthylcarbonyl)-L-lysin (Beispiel 3d) werden zusammen mit 225 mg (0,45 mmol) Terbiumcarbonat in 5 ml Wasser fünf Stunden bei 45°C gerührt. Anschließend wird filtriert, eingedampft und an Kieselgel RP-18 chromatographisch (Wasser/Acetonitril) gereinigt.
Ausbeute: 690 mg (89 % der Theorie) farbloser Feststoff.

| Analyse (bezogen auf lösungsmittelfreie Substanz): | | | | | | |
|---|---|---|---|---|---|---|
| ber. | C 41,78 | H 3,86 | N 6,50 | Na 5,33 | Tb 18,43 | O 24,11 |
| gef. | C 41,59 | H 3,97 | N 6,34 | Na 5,50 | Tb 18,26 | |

Die Verknüpfung mit einem erfindungsgemäßen Peptid erfolgt analog zu Beispiel 1d.

### Beispiel 4

N-Terminal verknüpftes Peptid-Konjugat mit dem Gadoliniumkomplex des Dinatriumsalzes von N,N-Bis-{2-[N',N'-bis-(carboxymethyl)-amino]-ethyl}-L-3-[(4-thiocarbonylamino)-phenyl]-alanin

### a) N,N-Bis-{2-[N',N'-bis-(carboxymethyl)-amino]-ethyl}-L-3-[(4-isothiocyanato)-phenyl]-alanin

155 mg (0,31 mmol) N,N-Bis-{2-[N',N'-bis-(carboxymethyl)-amino]-ethyl}-3-(4-aminophenyl)-alanin (JOC 58, 1151, 1993) werden in 5 ml Methanol gelöst und mit einer 0,2 normalen Lösung von Thiophosgen in Chloroform (1,7 ml, 0,34 mmol) versetzt und eine Stunde bei Raumtemperatur gerührt. Anschließend wird die Reaktionsmischung zur Trockne eingedampft, in Methanol aufgenommen und mit Aceton gefällt. Der Feststoff wird mit Aceton gewaschen und im Vakuum getrocknet.
Ausbeute: 0,16 g (95 % d. Th.) schwachgelber Feststoff.

| Analyse (bezogen auf lösungsmittelfreie Substanz): | | | | | |
|---|---|---|---|---|---|
| ber. | C 48,88 | H 5,22 | N 10,36 | S 5,93 | O 31,39 |
| gef. | C 48,65 | H 5,34 | N 10,23 | S 5,76 | |

### b) Gadoliniumkomplex des Dinatriumsalzes von N,N-Bis-{2-[N',N'-bis-(carboxymethyl)-amino]-ethyl -L-3-[(4-isothiocyanato)-phenyl]-alanin

100 mg (0,18 mmol) N,N-Bis-{2-[N',N'-bis-(carboxymethyl)-amino]-ethyl}-L-3-[(4-isothiocyanato)-phenyl]-alanin (Beispiel 4a) werden in 3 ml Methanol gelöst und mit 66 mg (0,18 mmol) Gadoliniumchlorid in 3 ml Methanol versetzt. Nach 30 Minuten neutralisiert man mit 0,1 normaler NaOH in Methanol, rührt weitere 30 Minuten und dampft anschließend zur Trockne ein. Der Rückstand wird in Methanol aufgenommen und mit Aceton gefällt. Die Fällung wird mit Aceton gewaschen, in Wasser aufgenommen, filtriert und lyophilisiert.
Ausbeute: 0,12 g (90 % d. Th.) farbloser Feststoff.

| Analyse (bezogen auf lösungsmittelfreie Substanz): | | | | | | | |
|---|---|---|---|---|---|---|---|
| ber. | C 35,77 | H 3,14 | N 7,58 | S 4,34 | Gd 21,29 | Na 6,22 | O 21,66 |
| gef. | C 35,59 | H 2,96 | N 7,45 | S 4,19 | Gd 21,11 | Na 5,96 | |

### c) N-Terminal verknüpftes Peptid-Konjugat mit dem Gadoliniumkomplex des Dinatriumsalzes von N,N-Bis-{2-[N',N'-bis-(carboxymethyl)-amino]-ethyl}-L-3-[(4-thiocarbonylamino)-phenyl]-alanin

74 mg (0,1 mmol) Gadoliniumkomplex des Dinatriumsalzes von N,N-Bis-{2-[N',N'-bis-(carboxymethyl)-amino]-ethyl}-L-3-[(4-isothiocyanato)-phenyl]-alanin (Beispiel 4b) werden in 1 ml Dimethylsulfoxid gelöst und bei 50°C mit 12 mg (0,01 mmol) dPhe-cyclo-[Cys-Phe-dTrp-Z-Lys-Thr-Cys]-Thr-OH umgesetzt. Nach drei Stunden fällt man das Produkt mit Ether, saugt die Fällung ab und wäscht den Feststoff mehrmals mit Ether. Das Zwischenprodukt wird in Wasser/Methanol aufgenommen und an Palladium auf Aktivkohle hydriert. Nach drei Stunden bei Raumtemperatur wird filtriert und das Filtrat gefriergetrocknet. Das Gadoliniumkomplex-Peptid-Konjugat kann zur Reinigung dialysiert werden oder per HPLC chromatographiert werden.
Ausbeute: 15 mg (85 % der Theorie).

| Analyse (bezogen auf wasserfreie Substanz): | | | | | | | |
|---|---|---|---|---|---|---|---|
| ber. | C 48,13 | H 4,95 | N 11,07 | Na 2,59 | Gd 8,87 | S 5,43 | O 18,96 |
| gef.: | C 48,01 | H 4,79 | N 10,86 | Na 2,43 | Gd 8,74 | S 5,30 | |

### Beispiel 5

N-Terminal verknüpftes Peptid-Konjugat mit dem Bismuthkomplex des Dinatriumsalzes von N,N-Bis-{2-[N',N'-bis-(carboxymethyl)-amino]-ethyl}-L-glycin-4-(thiocarbonylamino)-benzylamid

### a) N,N-Bis-{2-[N',N'-bis-(tert.-butyloxycarbonylmethyl)-amino]-ethyl}-L-glycin-4-nitrobenzylamid

0,98 g (1,6 mmol) N,N-Bis-{2-[N',N'-bis-(tert.-butyloxycarbonylmethyl)-amino]-ethyl}-L-glycin (US 5514810) werden in 3 ml N,N-Dimethylformamid gelöst und 200mg (1,74 mmol) N-Hydroxysuccinimid zugegeben. Man kühlt auf 0°C ab und addiert 360 mg (1,74 mmol) Dicyclohexylcarbodiimid. Es wird eine Stunde bei 0°C und vier Stunden bei Raumtemperatur gerührt. Man kühlt auf 0°C ab und tropft innerhalb von 10 Minuten eine Lösung aus 300 mg (1,74 mmol) 4-Nitrobenzylamin in 2 ml N,N-Dimethylformamid zu. Man rührt eine Stunde bei 0°C und anschließend über Nacht bei Raumtemperatur. Es wird zur Trockne eingedampft und der Rückstand in 20 ml Essigsäureethylester aufgenommen. Man filtriert vom ausgefallenen Harnstoff ab und wäscht das Filtrat mit 20 ml 5-proz. wäßriger Sodalösung. Die organische Phase wird über Magnesiumsulfat getrocknet und imVakuum zur Trockne eingedampft. Zur Reinigung wird an Kieselgel chromatographiert.
Ausbeute: 0,95 g (79 % d. Th.)

| Analyse (bezogen auf lösungsmittelfreie Substanz): | | | | |
|---|---|---|---|---|
| ber. | C 59,10 | H 8,18 | N 9,31 | O 23,41 |
| gef. | C 58,96 | H 8,27 | N 9,19 | |

### b) N,N-Bis-{2-[N',N'-bis-(tert.-butyloxycarbonylmethyl)-amino]-ethyl}-L-glycin-4-aminobenzylamid

0,8 g (1,1 mmol) N,N-Bis-{2-[N',N'-bis-(tert.-butyloxycarbonylmethyl)-amino]-ethyl}-L-glycin-4-nitrobenzylamid (Beispiel 5a) werden in 5 ml Ethanol gelöst und nach Zugabe von 0,08 g Palladium auf Aktivkohle (10% Palladium) bis zur beendeten Wasserstoffaufnahme hydriert. Anschließend wurde filtriert und das Filtrat vollständig eingedampft.
Ausbeute: 0,75 g (95 % d. Th.) gelbliches Öl.

| Analyse (bezogen auf lösungsmittelfreie Substanz): | | | | |
|---|---|---|---|---|
| ber. | C 61,56 | H 8,80 | N 9,70 | O 19,95 |
| gef. | C 61,39 | H 8,91 | N 9,54 | |

### c) N,N-Bis-{2-[N',N'-bis-(carboxymethyl)-amino]-ethyl}-L-glycin-4-aminobenzylamid

0,7 g (0,97 mmol) N,N-Bis-{2-[N',N'-bis-(tert.-butyloxycarbonylmethyl)-amino]-ethyl}-L-glycin-4-aminobenzylamid (Beispiel 5b) werden in 0,35 ml (5 mmol) Trifluoressigsäure gelöst und 10 Stunden bei Raumtemperatur gerührt. Zur Aufarbeitung verdünnt man mit Wasser und dampft zur Trockne ein. Dieser Vorgang wird mehrmals wiederholt. Anschließend wird eine wäßrige Lösung des Produktes über eine Anionenaustauschersäule gereinigt und die produkthaltigen Fraktionen eingedampft.
Ausbeute: 0,4 g (83 % d. Th.) farbloser Feststoff.

| Analyse (bezogen auf lösungsmittelfreie Substanz): | | | | |
|---|---|---|---|---|
| ber. | C 50,70 | H 6,28 | N 14,08 | O 28,94 |
| gef. | C 50,58 | H 6,34 | N 13,89 | |

### d) N,N-Bis-{2-[N',N'-bis-(carboxymethyl)-amino]-ethyl}-L-glycin-4-(isothiocyanato)-benzylamid

310 mg (0,61 mmol) N,N-Bis-{2-[N',N'-bis-(carboxymethyl)-amino]-ethyl}-L-glycin-4-aminobenzylamid (Beispiel 5c) werden in 10 ml Methanol gelöst und mit einer 0,2 normalen Lösung von Thiophosgen in Chloroform (3,4 ml, 0,68 mmol) versetzt und zwei Stunden bei Raumtemperatur gerührt. Anschließend wird die Reaktionsmischung zur Trockne eingedampft, in Methanol aufgenommen und mit Aceton gefällt. Der Feststoff wird mit Aceton gewaschen und im Vakuum getrocknet.
Ausbeute: 0,28 g (85 % d. Th.) schwachgelber Feststoff.

| Analyse (bezogen auf lösungsmittelfreie Substanz): | | | | | |
|---|---|---|---|---|---|
| ber. | C 48,97 | H 5,42 | N 12,98 | S 5,94 | O 26,69 |
| gef. | C 48,76 | H 5,55 | N 13,06 | S 5,81 | |

### e) Bismuthkomplex des Dinatriumsalzes von N,N-Bis-{2-[N',N'-bis-(carboxymethyl)-amino]-ethyl -L-glycin-4-(isothiocyanato)-benzylamid

200 mg (0,37 mmol) N,N-Bis-{2-[N',N'-bis-(carboxymethyl)-amino]-ethyl}-L-glycin-4-(isothiocyanato)-benzylamid (Beispiel 5d) werden in 8 ml Methanol gelöst und mit 117 mg (0,37 mmol) Bismuthchlorid in 8 ml Methanol versetzt. Nach 45 Minuten neutralisiert man mit 0,1 normaler NaOH in Methanol, rührt 20 Minuten und dampft anschließend zur Trockne ein. Der Rückstand wird in Methanol aufgenommen und mit Aceton gefällt. Die Fällung wird mit Aceton gewaschen, in Wasser aufgenommen, filtriert und lyophilisiert.
Ausbeute: 0,26 g (91,5 % d. Th.) farbloser Feststoff.

| Analyse (bezogen auf lösungsmittelfreie Substanz): | | | | | | | |
|---|---|---|---|---|---|---|---|
| ber. | C 34,43 | H 3,28 | N 9,12 | S 4,18 | Bi 27,23 | Na 3,00 | O 18,76 |
| gef. | C 34,31 | H 3,19 | N 8,98 | S 4,10 | Bi 27,07 | Na 2,76 | |

### f) N-Terminal verknüpftes Peptid-Konjugat mit dem Bismuthkomplex des Dinatriumsalzes von N,N-Bis-{2-[N',N'-bis-(carboxymethyl)-amino]-ethyl}-L-glycin-4-(thiocarbonylamino)-benzylamid

77 mg (0,1 mmol) Bismuthkomplex des Dinatriumsalzes von N,N-Bis-{2-[N',N'-bis-(carboxymethyl)-amino]-ethyl }-L-glycin-4-(isothiocyanato)-benzylamid (Beispiel 5e) werden in 1 ml Dimethylsulfoxid gelöst und bei 45°C mit 12 mg (0,01 mmol) dPhe-cyclo-[Cys-Phe-dTrp-Z-Lys-Thr-Cys]-Thr-OH umgesetzt. Nach vier Stunden fällt man das Produkt mit Ether, saugt die Fällung ab und wäscht den Feststoff mehrmals mit Ether. Das Zwischenprodukt wird in Wasser/Methanol aufgenommen und an Palladium auf Aktivkohle hydriert. Nach fünf Stunden bei Raumtemperatur wird filtriert und das Filtrat gefriergetrocknet. Das Gadoliniumkomplex-Peptid-Konjugat kann zur Reinigung dialysiert werden oder per HPLC chromatographiert werden.
Ausbeute: 14 mg (78 % der Theorie).

| Analyse (bezogen auf wasserfreie Substanz): | | | | | | | |
|---|---|---|---|---|---|---|---|
| ber. | C 47,36 | H 4,98 | N 11,67 | Na 1,28 | Bi 11,61 | S 5,34 | O 17,77 |
| gef. | C 47,22 | H 5,07 | N 11,73 | Na 0,99 | Bi 11,40 | S 5,22 | |

### Beispiel 6

N-Terminal verknüpftes Peptid-Konjugat mit dem Europiumkomplex des Natriumsalzes von 1,4,7,10-Tetraazacyclododecan-1-[4-(carboxymethoxy)-phenyl]-essigsäure-4,7,10-triessigsäure

### a) 4-[O-(Ethoxycarbonylmethyl)-hydroxy]-phenylessigsäuremethylester

Zu 22,2 g (133 mmol) 4-Hydroxyphenylessigsäuremethylester in 150 ml Aceton addiert man 23,5 g (170 mmol) Kaliumcarbonat und versetzt unter Rückfluß mit 26,8 g (160 mmol) Bromessigsäureethylester. Nach einer Stunde gibt man 5,9 g (42,5 mmol) Kaliumcarbonat und 5,5 g (33 mmol) 4-Hydroxyphenylessigsäuremethylester zu und rührt anschließend weitere zwei Stunden am Rückfluß. Man filtriert den Feststoff ab, dampft das Lösemittel ab und destilliert den Rückstand im Vakuum.
Ausbeute: 29,5 g (88 % d. Th.) farbloses Öl.

| Analyse (bezogen auf lösungsmittelfreie Substanz): | | | |
|---|---|---|---|
| ber. | C 61,90 | H 6,39 | O 31,71 |
| gef. | C 61,73 | H 6,47 | |

### b) 4-[O-(Ethoxycarbonylmethyl)-hydroxy]-phenyl-2-brom-essigsäuremethylester

14,0 g (55 mmol) 4-[O-(Ethoxycarbonylmethyl)-hydroxy]-phenylessigsäuremethylester (Beispiel 6a) werden in 70 ml Tetrachlorkohlenstoff vorgelegt, mit 9,9 g (55 mmol) N-Bromsuccinimid und 100 mg (0,2 mmol) Benzoylperoxid versetzt. Nach fünfstündigem Kochen unter Rückfluß wird die Reaktionsmischung auf Raumtemperatur abgekühlt, zweimal mit gesättigter Natriumhydrogencarbonatlösung extrahiert, mit Wasser gewaschen und über Natriumsulfat getrocknet. Nach Filtration und Eindampfen erhält man das Produkt als schwachgelbes Öl.
Ausbeute: 18,1 g (99 % d. Th.)

| Analyse (bezogen auf lösungsmittelfreie Substanz): | | | | |
|---|---|---|---|---|
| ber. | C 47,15 | H 4,57 | Br 24,13 | O 24,16 |
| gef. | C 47,03 | H 4,68 | Br 23,96 | |

### c) 1,4,7,10-Tetraazacyclododecan-1-[4-(ethoxycarbonylmethoxy)-phenyl]-essigsäuremethylester

17,6 g (53 mmol) 4-[O-(Ethoxycarbonylmethyl)-hydroxy]-phenyl-2-bromessigsäuremethylester (Beispiel 6b) werden in 120 ml Chloroform gelöst und unter Eiskühlung mit 27,4 g (159 mmol) Cyclen versetzt. Man rührt über Nacht bei Raumtemperatur, wäscht viermal mit Wasser, trocknet die organische Phase, filtriert und dampft zur Trockne ein.
Ausbeute: 21,6 g (96 % d. Th.)

| Analyse (bezogen auf lösungsmittelfreie Substanz): | | | | |
|---|---|---|---|---|
| ber. | C 59,70 | H 8,11 | N 13,26 | O 18,93 |
| gef. | C 59,58 | H 8,05 | N 13,39 | |

### d) 1,4,7,10-Tetraazacyclododecan-1-[4-(ethoxycarbonylmethoxy)-phenyl]-essigsäuremethylester-4,7,10-triessigsäure-tert.-butylester

21,0 g (50 mmol) 1,4,7,10-Tetraazacyclododecan-1-[4-(ethoxycarbonylmethoxy)-phenyl]-essigsäuremethylester (Beispiel 6c) werden in 160 ml Acetonitril gelöst. Man gibt 18,2 g (170 mmol) Natriumcarbonat hinzu und addiert tropfenweise 33,5 g (170 mmol) Bromessigsäure-tert.-butylester. Nach drei Stunden Rühren bei 60°C saugt man ab, engt die Lösung ein und chromatographiert den Rückstand an Kieselgel.
Ausbeute: 23,9 g (62 % d. Th.)

| Analyse (bezogen auf lösungsmittelfreie Substanz): | | | | |
|---|---|---|---|---|
| ber. | C 61,24 | H 8,43 | N 7,32 | O 23,01 |
| gef. | C 61,14 | H 8,50 | N 7,18 | |

### e) 1,4,7,10-Tetraazacyclododecan-1-[4-(ethoxycarbonylmethoxy)-phenyl]-essigsäuremethylester-4,7,10-triessigsäure

13,8 g (18 mmol) 1,4,7,10-Tetraazacyclododecan-1-[4-(ethoxycarbonylmethoxy)-phenyl]-essigsäuremethylester-4,7,10-triessigsäure-tert.-butylester (Beispiel 6d) werden in 80 ml Anisol mit 200 ml Trifluoressigsäure umgesetzt. Man rührt über Nacht bei Raumtemperatur und zwei Stunden bei 60°C. Anschließend engt man ein und destilliert mehrmals mit Wasser nach.
Ausbeute: 9,8 g (92 % d. Th.)

| Analyse (bezogen auf lösungsmittelfreie Substanz): | | | | |
|---|---|---|---|---|
| ber. | C 54,72 | H 6,12 | N 9,45 | O 29,70 |
| gef. | C 54,83 | H 6,07 | N 9,36 | |

### f) 1,4,7,10-Tetraazacyclododecan-1-[4-(carboxymethoxy)-phenyl]-essigsäure-4,7,10-triessigsäure, Pentakaliumsalz

9,5 g (16 mmol) 1,4,7,10-Tetraazacyclododecan-1-[4-(ethoxycarbonylmethoxy)-phenyl]-essigsäuremethylester-4,7,10-triessigsäure (Beispiel 6e) werden in 30 ml Methanol gelöst, mit 4,5 g Kaliumhydroxid in 30 ml Wasser gelöst versetzt und vier Stunden bei Raumtemperatur gerührt. Anschließend kocht man drei Stunden am Rückfluß. Man zieht das Methanol ab und setzt das Rohprodukt in die nächste Stufe ohne weiteren Reinigungsschritt ein.
Rohausbeute: 11,8 g (99 % d. Th.)

### g) Europiumkomplex des Natriumsalzes von 1,4,7,10-Tetraazacyclododecan-1-[4-(carboxymethoxy)-phenyl]-essigsäure-4,7,10-triessigsäure

10,4 g (14 mmol) 1,4,7,10-Tetraazacyclododecan-1-[4-(carboxymethoxy)-phenyl]-essigsäure-4,7,10-triessigsäure, Pentakaliumsalz (Beispiel 6f) werden in 40 ml Wasser gelöst und mit 3,4 g (7 mmol) Europiumcarbonat versetzt. Man addiert 5,7 ml Essigsäure und refluxiert über Nacht. Nach beendeter Komplexierung neutralisiert man mit zweinormaler Natronlauge, reinigt das Rohprodukt über eine Ionenaustauschersäule und lyophilisiert die produkthaltigen Fraktionen.
Ausbeute: 9,8 g (94 % der Theorie) farbloses Lyophilisat.

| Analyse (bezogen auf wasserfreie Substanz): | | | | | | |
|---|---|---|---|---|---|---|
| ber. | C 38,57 | H 3,91 | N 7,50 | Na 6,15 | Eu 20,33 | O 23,55 |
| gef. | C 38,43 | H 4,02 | N 7,28 | Na 5,97 | Eu 20,21 | |

### h) N-Terminal verknüpftes Peptid-Konjugat mit dem Europiumkomplex des Natriumsalzes von 1,4,7,10-Tetraazacyclododecan-1-[4-(carboxymethoxy)-phenyl]-essigsäure-4,7,10-triessigsäure

0,1 g (0,13 mmol) Europiumkomplex des Natriumsalzes von 1,4,7,10-Tetraazacyclododecan-1-[4-(carboxymethoxy)-phenyl]-essigsäure-4,7,10-triessigsäure (Beispiel 6g) werden in 1 ml Dimethylsulfoxid gelöst und bei 50°C mit 48 mg (0,15 mmol) O-(Benzotriazol-1-yl)-N,N,N',N'-tetramethyluroniumtetrafluoroborat (TBTU) umgesetzt. Nach einer Stunde kühlt man auf Raumtemperatur ab und setzt mit 9,5 mg (5 *µ*mol) Ala-Gly-cyclo-[Cys-Z-Lys-Asn-Phe-Phe-Trp-Z-Lys-Thr-Phe-Thr-Ser-Cys]-OH um. Nach 3 Stunden fällt man das Produkt mit Ether, saugt die Fällung ab und wäscht den Feststoff mehrmals mit Ether. Das Zwischenprodukt wird in Wasser/Methanol aufgenommen und an Palladium auf Aktivkohle hydriert. Nach drei Stunden bei Raumtemperatur wird filtriert und das Filtrat gefriergetrocknet. Das Europiumkomplex-Peptid-Konjugat kann zur Reinigung dialysiert werden oder per HPLC chromatographiert werden.
Ausbeute: 10,6 mg (90 % der Theorie)

| Analyse (bezogen auf wasserfreie Substanz): | | | | | | | |
|---|---|---|---|---|---|---|---|
| ber. | C 48,79 | H 5,00 | N 10,27 | Na 2,59 | Eu 8,97 | S 3,62 | O 20,76 |
| gef. | C 48,62 | H 4,88 | N 9,98 | Na 2,31 | Eu 8,82 | S 3,50 | |

### Beispiel 7

Cystein-verknüpftes Peptid-Konjugat mit dem Terbiumkomplex der 1,4,7,10-Tetraazacyclododecan-1-[4-(4-aza-6-thio-5-oxo-hexylaminocarbonylmethoxy)-phenyl]-essigsäure-4,7,10-triessigsäure

### a) [3-[N-(tert.-Butoxycarbonyl)amino]propyl]-N'-(bromacetyl)-amid

2,5 g (14,4 mmol) [3-[N-(tert.-Butoxycarbonyl)amino]propyl]amin werden in 15 ml Dioxan gelöst und nach Zugabe von 4,4 ml Triethylamin bei 0°C mit 3,2 g (16 mmol) Bromacetylbromid versetzt. Man rührt über Nacht bei Raumtemperatur und addiert anschließend weitere 320 mg Bromacetylbromid. Nach zwei Stunden bei Raumtemperatur wird der Niederschlag abgesaugt, die Lösung eingeengt und der Rückstand in Essigsäureethylester aufgenommen. Man wäscht mit Wasser und trocknet die organische Phase über Natriumsulfat.
Ausbeute: 3,2 g (75 % d. Th.)

| Analyse (bezogen auf lösungsmittelfreie Substanz): | | | | | |
|---|---|---|---|---|---|
| ber. | C 40,69 | H 6,49 | Br 27,07 | N 9,49 | O 16,26 |
| gef. | C 40,50 | H 6,37 | Br 26,89 | N 9,58 | |

### b) [3-[N-( Bromacetyl)amino]propyl]amin, Hydrochlorid

3,1 g (10,5 mmol) [3-[N-(tert.-Butoxycarbonyl)amino]propyl]-N'-(bromacetyl)-amid (Beispiel 7a) werden mit 50 mmol 1M Salzsäure in Essigsäureethylester für fünf Stunden bei Raumtemperatur gerührt. Man saugt das Produkt ab und wäscht den Feststoff mit Essigsäureethylester nach.
Ausbeute: 2,3 g (95 % d. Th.)

| Analyse (bezogen auf lösungsmittelfreie Substanz): | | | | | | |
|---|---|---|---|---|---|---|
| ber. | C 25,94 | H 5,22 | Cl 15,31 | Br 34,51 | N 12,10 | O 6,91 |
| gef. | C 25,76 | H 5,41 | Cl 15,55 | Br 34,34 | N 11,97 | |

### c) Terbiumkomplex der 1,4,7,10-Tetraazacyclododecan-1-[4-(4-aza-6-brom-5-oxohexylaminocarbonylmethoxy)-phenyl]-essigsäure-4,7,10-triessigsäure

1,5 g (1,95 mmol) Terbiumkomplex des Natriumsalzes von 1,4,7,10-Tetraazacyclododecan-1-[4-(carboxymethoxy)-phenyl]-essigsäure-4,7,10-triessigsäure (hergestellt in Analogie zu Beispiel 6g) werden in 10 ml Dimethylsulfoxid gelöst und bei 60°C mit 720 mg (2,25 mmol) O-(Benzotriazol-1-yl)-N,N,N',N'-tetramethyluronium-tetrafluoroborat (TBTU) umgesetzt. Nach einer Stunde kühlt man auf Raumtemperatur ab und versetzt mit 522 mg (2,25 mmol) [3-[N-( Bromacetyl)amino]propyl]amin, Hydrochlorid (Beispiel 7b) und 0,63 ml (4,5 mmol) Triethylamin. Nach sechs Stunden fällt man das Produkt mit Ether, saugt die Fällung ab und wäscht den Feststoff mehrmals mit Ether.
Ausbeute: 1,3 g (75 % der Theorie)

| Analyse (bezogen auf wasserfreie Substanz): | | | | | | |
|---|---|---|---|---|---|---|
| ber. | C 39,25 | H 4,54 | Br 9,00 | N 9,47 | Tb 17,91 | O 19,83 |
| gef. | C 39,09 | H 4,66 | Br 8,83 | N 9,54 | Tb 17,68 | |

### d) Cystein-verknüpftes Peptid-Konjugat mit dem Terbiumkomplex der 1,4,7,10-Tetraazacyclododecan-1-[4-(4-aza-6-thio-5-oxo-hexylaminocarbonylmethoxy)-phenyl]-essigsäure-4,7,10-triessigsäure

0,33 mg (0,1 *µ*mol) H-His-Ser-Asp-Ala-Val-Phe-Tyr-Asp-Asn-Tyr-Thr-Arg-Leu-Arg-Lys-Glu-Cys-Ala-Val-Lys-Lys-Tyr-Leu-Asn-Ser-Ile-Leu-Asn-OH werden in 0,1 ml wasserfreiem N,N-Dimethylformamid gelöst und mit 0,244 mg (0,75 *µ*mol) Cäsiumcarbonat versetzt. Nach zehn Minuten gibt man 0,887 mg (1 *µ*mol) Terbiumkomplex der 1,4,7,10-Tetraazacyclododecan-1-[4-(4-aza-6-brom-5-oxohexylaminocarbonylmethoxy)-phenyl]-essigsäure-4,7,10-triessigsäure (Beispiel 7c) hinzu und läßt eine Stunde bei 40°C rühren. Das Terbiumkomplex-Peptid-Konjugat kann zur Reinigung dialysiert werden oder per HPLC chromatographiert werden.
Ausbeute: 0,29 mg (71 % der Theorie)

| Analyse (bezogen auf wasserfreie Substanz): | | | | | | |
|---|---|---|---|---|---|---|
| ber. | C 50,92 | H 6,70 | N 17,06 | Tb 3,87 | S 0,78 | O 20,66 |
| gef. | C 50,86 | H 6,81 | N 16,92 | Tb 3,59 | S 0,64 | |

### Example 8

### Synthesis of ligands

### General Material and methods

All reagents were obtained from commercial suppliers and used without further purification. NMR spectra were recorder on Bruker Ac-200 MHz or 500 MHz spectrometer equipped with a multi-nuclear quad probe (¹H, ¹³C, ³¹P and ¹⁹F) at 297° K. ¹H spectra in D₂O were recorded by employing solvent suppression pulse sequence. Melting points were determined by capillary melt methods and were uncorected.

### Synthetic procedures

1,7-Bis(benzyloxycarbonyl)-1,4,7,10-tetraazacyclododecane (1) and 1,7-Bis(benzyloxycarbonyl)-1,4,7,10-tetraazacyclododecane-bis(methanephosphonic acid diethyl ester) (2) were prepared according to the literature.¹

### a) Synthesis of 1,4,7,10-Tetraazacyclododecane-1,7-bis-(methanephosphonic acid diethyl ester)

A solution of 5g (6.75 mmol) of 1,7-Bis(benzyloxycarbonyl)-1,4,7,10-tetraazacyclododecane bis(methanephosphonic acid diethyl ester) in absolute ethanol was mixed with 6.84 ml (67.50 mmol) cyclohexene and 1g of 5% Pd/C catalyst was added. The mixture was stirred under reflux 2h, filtered from catalyst washed with ethanol, and the combined filtrates were evaporated under vaccum. The residue is a pale yellow oil; 3.18g, 100%.
¹H NMR and ¹³C NMR conformable to literature. ¹
¹ Z. Kovacs, A.D. Sherry, *Synthesis, 759,* (1996)
Z. Kovacs and A.D. Sherry,*J. Chem. Soc. Chem. Commun.,* 185, (1995)

### b) Synthesis of 1-(6-Fluoro-2-quinolinemethyl)-1,4,7,10-tetraazacyclododecane-4,10-bis(methanephosphonic acid diethyl ester)

1,4,7,10-Tetraazacyclodoecane-1,7-bis(methanephosphonic acid diethyl ester) (10mmole) was dissolved in 150 ml of dry acetonitrile and solution of 2-Chloromethylene-6-fluoro-quinoline (10 mmole) in acetonitrile was added dropwise. The mixture was stirred at 40 C for two days. Solvent was removed under vacuum. The residue was purified on silica gel column using solvent system (10:2:1) dioxane: methanol: ammonium hydroxide. Yield 25% of monoalkylated.
¹H NMR (500MHz, CDCl₃) δ: 1.16 (t, J= 7.00 Hz, 3H, CH₃), 2.74 (br d,J=8.41 Hz,12H, NCH₂CH₂N), 2.89 (br, 8H, NCH₂CH₂N and PCH₂N), 3.86 (d, J-2.41 Hz, 4H, CH₂Ar), 3.93 (q, J=7.03 Hz, 8H, OCH₂), 7.24-7.42 (m, 4H, Ar), 7.84 (d J=8.62 Hz 2H, Ar), 8.05-8.09 (m, 4H, Ar); ¹³C NMR (300MHz, CDCl₃) δ: 162.04 (Ar), 158.76 (Ar), 144.72 (Ar), 135.58 (d, J=20.04 Hz, Ar), 131.52 (d, J=35.70 Hz, Ar), 128.05 (d, J= 39.28 Hz Ar), 122.51 (Ar), 119.13 (d, J=102.30 Hz, Ar), 110.54 (d, J= 86.1 Hz, Ar), 61.63 (POCH₂), 60.10 ( CH₂Ar), 53.63 (d, J=23.39 Hz, PCH₂N), 52.02 (NCH₂CH₂N), 49.98 (NCH₂CH₂N), 16.46 (CH₃); ³¹P NMR (300MHz, CDCl₃) δ: 26.26.

### c) 1-(6-Fluoro-2-quinolinemethyl)-7-(methanecarboxylic acid ethyl ester)-1,4,7,10-tetraazacyclododecane-4,10-bis(methanephosphonic acid diethyl ester)

1-(6-Fluoro-2-quinolinemethyl)-1,4,7,10-tetraazacyclododecane-4,10-bis(methanephosphonic acid diethyl ester) (10 mmol) was dissolved in dry acetonitrile and ethyl bromoacetate (10% excess) and anhydrous K₂CO₃ (11 mmole) were added. The solution was stirred at 40 C a few hours. Subsequently the solvent was removed under vacuum. The residue was dissolved in dichloromethane and purified on silica gel column eluting with dioxane: methanol: ammonium hydroxide (10:2:1). After concentration of eluent, the product was isolated as a thick pale yellow liquid.
Yield 78%.
¹H NMR (500MHz, CDCl₃) δ: 1.14-1.19 (m, 15H, CH₃), 2.71-2.88 (br 20H, NCH₂CH₂N, PCH₂N), 3.42 (s, 2H, NCH₂CO), 3.76 9s, 2H, CH₂Ar), 3.98 (m, 10H, OCH₂), 7.32-7.45 (m, 2H, Ar), 7.78 (d, J=8.02 Hz 1H, Ar), 7.96-8.08 (m, 2H, Ar); ¹³C NMR (300MHz, CDCl₃) δ:171.57 (COOEt), 162.03 (Ar), 158.75 (Ar), 144.71 (Ar), 135.58 (d, J=20.04 Hz, Ar), 131.52 (d, J=60.01 Hz, Ar), 128.05 (d, J= 39.60 Hz Ar), 122.84 (Ar), 119.36 (d, J=102.30 Hz, Ar), 110.53 (d, J= 86.41 Hz, Ar), 62.48 (CH₂Ar), 61.62 (d, J=30.01 Hz, OCH2) 60.10 ( OCH₂), 55.63 (d, J=23.39 Hz, PCH₂N), 52.71 (NCH₂CH₂N), 52.02 (NCH₂CH₂N), 49.97 (NCH₂CH₂N), 16.46 (d, J=22.08 Hz, CH₃); ³¹P NMR (300MHz, CDCl₃) δ: 26.26.

### d) Synthesis of 1-(6-Fluor-2-quinolinemethyl)-7-(methanecarboxylic acid)-1,4,7,10-tetraazacyclododecane- 4,10-bis(methanephosphonic acid)

1-(6-Fluor-2-quinolinemethyl)-7-(methanecarboxylic acid ethyl ester)-4,10-bis(methylenephosphonic acid diethyl ester)-1,4,7,10-tetraazacyclododecane (5 mmol) was dissolved in 6M hydrochloric acid. The solution was refluxed for two days. Hydrochloric acid was removed under vacuum by azeotropic destillation. The residue was purified on anion-exchange column Q- Sepharosc,™ eluting first with deionized water, then with 1M hydrochloric acid. Follow freeze-drying of the eluent, the product was isolated as white solid and further characterized by:
¹H NMR
¹³C NMR
³¹P NMR
Yield 96%

### e) Synthesis of 1-(6-Chloro-2-quinolinemethyl)-4,10-bis(benzyloxycarbonyl)-1,4,7,10-tetraazacyclododecane

1,7-Bis-(benzyloxycarbonyl)-1,4,7,10-tetraazacyclododecane (10mmole) was dissolved in 150 ml of dry acetonitrile and solution of 6-Chloro-2-chloromethylene-quinoline (2-Chloromethylene-6-fluoro-quinoline) (10 mmole) in acetonitrile was added dropwise. The mixture was stirred at 40 C for three days. Solvent was removed under vacuum. The residue was purified on silica gel column using solvent system 10:4:1 (ethyl acetate : Methanol : ammonium hydroxide). Yield 75%.
¹H NMR (500MHz, CDCl₃) δ: 3.07-3.47 (br 8H, NCH₂CH₂N), 3.58-3.74 (br, 4H, NCH₂CH₂N), 3.98-4.06 (br, 4H, NCH₂CH₂N), 4.13 (s, 2H, CH₂Ar), 4.27-4.49 (br, 4H, OCH₂Ar), 6.95-7.23 (m, 7H, Ar), 7.27-7.38 (m, 4H, Ar), 7.52-7.57 (m. 1H, Ar), 7.64-7.75 (m, 2H, Ar), 7.90-7.96 (m, 1H, Ar); ¹³C NMR (500MHz, CDCl₃) δ: 167.63 (Ar), 159.59 (Ar), 156.10 (m, NCOO), 145.96 (d, J=30.15 Hz, Ar), 135.75-135.29 (m, Ar), 130.47 (d J=16.49 Hz, Ar), 128.48 (d, J= 29.49 Hz, Ar), 128.30 (Ar), 128.12 (d, J=51.49 Hz, Ar), 127.83 ( d, J=115.47 Hz, Ar), 127.49 (d, J= 33.02 Hz Ar), 126.30 (Ar), 121.68 (Ar), 67.25 (d, J=66.06 Hz, OCH₂Ar), 66.13 (CH₂Ar), 52.86 (NCH₂CH₂N), 48.15 (NCH₂CH₂N), 45.06 (HNCH₂CH₂N).

### f) 1-(6-Chloro-2-quinolinemethyl)-4,10-bis(benzyloxycarbonyl)-7-(methanecarboxylic acid ethyl ester)-1,4,7,10-tetraazacyclododecane

1,7-Bis(benzyloxycarbonyl)-4-(6-chloro-2-quinolinemethyl)-1,4,7,10-tetraazacyclododecane was dissolved in dry acetonitrile and ethyl bromoacetate and di-ipropyle-ethylamine was added. The solution was stirred at 40 C a few houres. The mixture was evaporated to dryness. The residue was dissolved in ethyl acetate. After a few minutes di-ipropyle-ethylamine hydrochloride was filtered off, washed with ethyl acetate. After evaporation of the solvent under vacuum, the residue was purified on silica gel column eluting with ethyl acetate. Yield 78%.
¹H NMR (500MHz, CDCl₃) δ: 1.14-1.19 (m, 15H, CH₃), 2.71-2.88 (br, 20H, NCH₂CH₂N, PCH₂N), 3.42 (s,2H, NCH₂CO), 3.7 (br, 4H, NCH₂CH₂N), 3.98-4.06 (br, 4H, NCH₂CH₂N), 4.13 (s, 2H, CH₂Ar), 4.27-4.49 (br, 4H, OCH₂Ar), 6.95-7.23 (m, 7H, Ar), 7.27-7.38 (m, 4H, Ar), 7.52-7.57 (m. 1H, Ar), 7.64-7.75 (m, 2H, Ar), 7.90-7.96 (m, 1H, Ar); ¹³C NMR (500MHz, CDCl₃) δ: 167.63 (Ar), 159.59 (Ar), 156.10 (m, NCOO), 145.96 (d, J=30.15 Hz, Ar), 135.75-135.29 (m, Ar), 130.47 (d J=16.49 Hz, Ar), 128.48 (d, J= 29.49 Hz, Ar), 128.30 (Ar), 128.12 (d, J=51.49 Hz, Ar), 127.83 ( d, J=115.47 Hz, Ar), 127.49 (d, J= 33.02 Hz Ar), 126.30 (Ar), 121.68 (Ar), 67.25 (d, J=66.06 Hz, OCH₂Ar), 66.13 (CH₂Ar), 52.86 (NCH₂CH₂N), 48.15 (NCH₂CH₂N), 45.06 (HNCH₂CH₂N).

### g) 1-(6-Chloro-2-quinolinemethyl)-7-(methanecarboxylic acid ethyl ester)-1,4,7,10-tetraazacyclododecane

1,7-Bis(benzyloxycarbonyl)-4-(6-chloro-2-quinolinemethy)-10-(ethyl methanecarboxylic acid)-1,4,7,10-tetraazacyclododecane was dissolved in ethanole, and 5% Pd/C and cyclohexene were added. Mixture was stirring under reflux for one hour. The catalyst was filtered off and washed with ethanol. The solvent was removed under vacuum. Yield 80%
¹H NMR (500 MHz, CDCl₃) δ: 1.17 (t, J=7.20 Hz, 3H, CH₃)2.64 (br, 9H, HNCH₂CH₂NH), 2.82 (br, 9H, NCH₂CH₂N), 3.46 (s, 2H, NCH₂CO), 3.98 (s, 2H, CH₂Ar), 4.06 (q, J=7.22 Hz, 2H, OCH₂), 7.40 (t, J=8.02 Hz, 1H, Ar), 7.54-7.62 (m, 1H, Ar), 7.69 (d, J=7.20 Hz, 1H, Ar), 7.91 (d J=8.20 Hz, 1H, Ar), 8.02 (d, J= 8.46 Hz, 1H, Ar); ¹³C NMR (500 MHz, CDCl₃) δ: 171.48 (COOEt), 159.87,147.21, 136.29, 129.29, 128.74,127.41, 127.14, 126.10, 121.35 (quinoline carbones), 63.47 (OCH₂), 60.35 (CH₂Ar), 56.14 (NCH₂CO), 52.37 (NCH₂CH₂N), 51.42 (NCH₂CH₂N), 46.79 (d, J=69.5 Hz, HNCH₂CH₂N)

**Alkylation:** 1-(6-Chloro-2-quinolinemethyl)-7-(ethyl methanecarboxylic acid)-1,4,7,10-tetraazacyclododecane was dissolved in triethyl phosphite and paraformaldehyde was added. The mixture was stirred for three days. The volatile impurities were removed under vacuum. The residue was purified on silica gel column. Yield 64%.

**Hydrolysis:** 1-(6-Chloro-2-quinolinemethyl)-7-(ethyl methanecarboxylic acid)-4,10-bis(methylenephosphonic acid diethyl ester)-1,4,7,10-tetraazacyclododecane was dissolved in 6M hydrochloric acid. The solution was refluxed for two days.

Hydrochloric acid was removed under vacuum by azeotropic destillation. The residue was purified on ion-exchange column. Yield 96%.

## Patentansprüche

1. Verbindungen der allgemeinen Formel (I)
A¹ - L¹ - (X)ₘ - L² - A² (I)
worin
X für eine α, β oder γ-Aminosäure mit D oder L-Konfiguration und
m für eine Zahl von 5 bis 30 steht,
wobei die resultierende Aminosäuresequenz (X)ₘ geradkettiger Natur oder über eine Disulfidbrücke zwischen zwei Cysteinen oder Homocysteinen oder amidisch zwischen N- und C-Terminus cyclisiert sein kann und für die Aminosäuresequenz des vasoaktiven intestinalen Peptids (VIP), des Somatostatins oder des Neurotensins, oder für Fragmente, Teilsequenzen, Derivate oder Analoga des VIP, des Somatostatins oder des Neurotensins steht,
A¹ für ein Wasserstoffatom, eine offenkettige oder cyclische Polyaminopolycarbonsäure oder Polyaminopolyphosphonsäure steht, welche eine Arylgruppe oder einen Heteroaromaten enthält und ein Metallatom der Ordnungszahlen 57 bis 83 komplexiert,
L¹ und L² unabhängig voneinander einen Acetylrest oder einen Alkylrest mit bis zu 10 C-Atomen, der gegebenenfalls mit 1 bis 3 Carboxygruppen und/oder 1 bis 6 Hydroxygruppen und/oder 1 bis 6 Amidgruppen substituiert sein kann, oder einen Poly(oxyethylen)rest mit 2 bis 30 -CH₂CH₂O-Einheiten darstellen,
A² für eine Hydroxygruppe, eine Aminogruppe, eine offenkettige oder cyclische Polyaminopolycarbonsäure oder Polyaminopolyphosphonsäure steht, welche eine Arylgruppe oder einen Heteroaromaten enthält und ein Metallatom der Ordnungszahlen 57 bis 83 komplexiert,
unter der Bedingung, daß mindestens einer der Reste A¹ oder A² eine offenkettige oder cyclische Polyaminopolycarbonsäure oder Polyaminopolyphosphonsäure darstellt, welche eine Arylgruppe oder einen Heteroaromaten enthält und ein Metallatom der Ordnungszahlen 57 bis 83 komplexiert,
wobei für den Fall, daß A¹ und/oder A² eine offenkettige oder cyclische Polyaminopolycarbonsäure oder Polyaminopolyphosphonsäure darstellen, welche eine Arylgruppe oder einen Heteroaromaten enthält und ein Metallatom der Ordnungszahlen 57 bis 83 komplexiert, A¹ an die N-terminale Aminogruppe und A² an eine Aminogruppe der Aminosäure Lysin oder an eine Hydroxygruppe der Aminosäure Serin oder an die Mercaptogruppe der Aminosäure Cystein oder Homocystein in beliebiger Position innerhalb der Aminosäuresequenz (X)ₘ geknüpft ist,
und deren physiologisch verträgliche Salze.

2. Verbindungen nach Anspruch 1, **dadurch gekennzeichnet, daß** die offenkettige Polyaminopolycarbonsäure, welche eine Arylgruppe oder einen Heteroaromaten enthält und ein Metallatom der Ordnungszahlen 57 bis 83 komplexiert, ein Derivat der Diethylentriaminpentaessigsäure (DTPA) ist.

3. Verbindungen nach Anspruch 1, **dadurch gekennzeichnet, daß** die cyclische Polyaminopolycarbonsäure, welche eine Arylgruppe oder einen Heteroaromaten enthält und ein Metallatom der Ordnungszahlen 57 bis 83 komplexiert, ein Derivat von 1,4,7,10-Tetraazacyclododecan (DOTA) ist.

4. Verbindungen nach Anspruch 1, **dadurch gekennzeichnet, daß** die offenkettige Polyaminopolycarbonsäure ein Molekül gemäß allgemeiner Formel (II) ist: 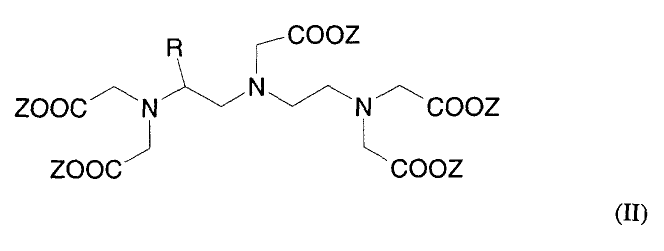 worin Z unabhängig voneinander für ein Wasserstoffatom oder ein Metallionenäquivalent eines Elementes der Ordnungszahlen 57 bis 83 und R für einen offenkettigen oder cyclischen, verzweigten oder unverzweigten C₁-C₁₀-Alkylrest steht, welcher mindestens einen aromatischen Ring sowie gegebenenfalls 1 bis 5 Sauerstoffatome, 1 bis 3 Carboxygruppen und/oder 1 bis 3 Amidgruppen enthält.

5. Verbindungen nach Anspruch 4, **dadurch gekennzeichnet, daß** R für einen der folgenden Reste steht: 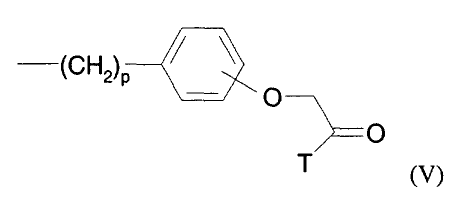 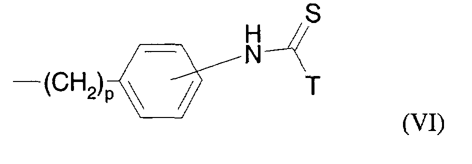 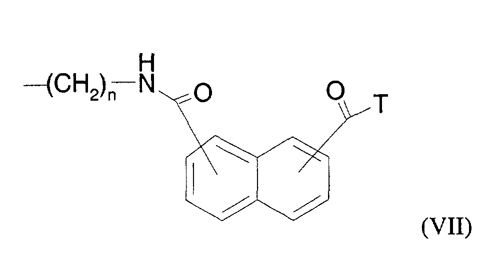 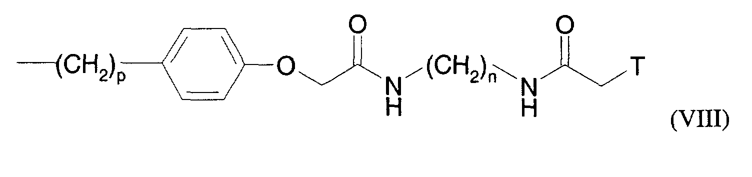 worin T jeweils die Verknüpfungsstelle mit dem Peptid darstellt, p für eine Zahl 0 oder 1 und n für eine Zahl zwischen 2 und 6 steht.

6. Verbindungen nach Anspruch 1, **dadurch gekennzeichnet, daß** die offenkettige Polyaminopolycarbonsäure ein Molekül gemäß allgemeiner Formel (III) ist: 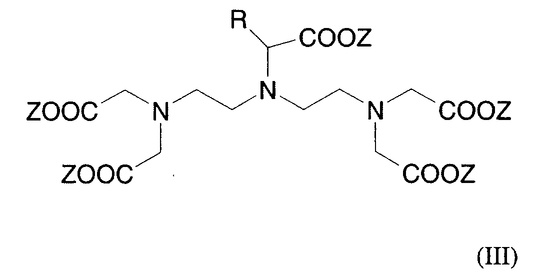 worin Z unabhängig voneinander für ein Wasserstoffatom oder ein Metallionenäquivalent eines Elementes der Ordnungszahlen 57 bis 83 und R für einen offenkettigen oder cyclischen, verzweigten oder unverzweigten C₁-C₁₀-Alkylrest steht, welcher mindestens einen aromatischen Ring sowie gegebenenfalls 1 bis 5 Sauerstoffatome, 1 bis 3 Carboxygruppen und/oder 1 bis 3 Amidgruppen enthält.

7. Verbindungen nach Anspruch 6, **dadurch gekennzeichnet, daß** der Rest R für einen der folgenden Reste steht: 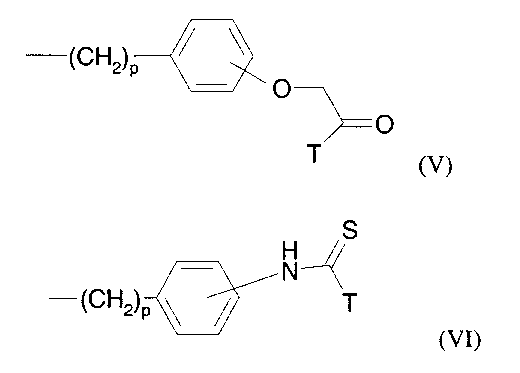 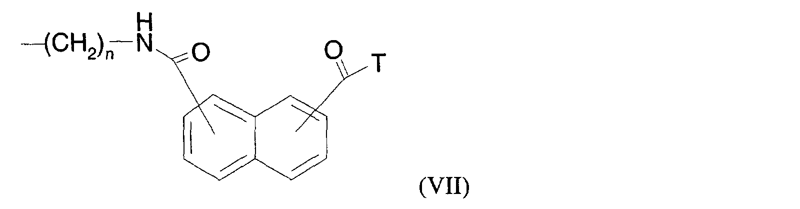 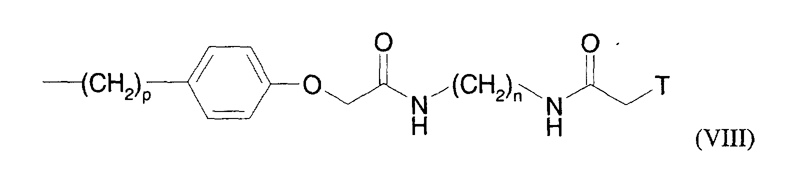 worin T jeweils die Verknüpfungsstelle mit dem Peptid darstellt, p für eine Zahl 0 oder 1 und n für eine Zahl zwischen 2 und 6 steht.

8. Verbindungen nach Anspruch 1, **dadurch gekennzeichnet, daß** die offenkettige Polyaminopolycarbonsäure ein Molekül gemäß allgemeiner Formel (IV) ist: 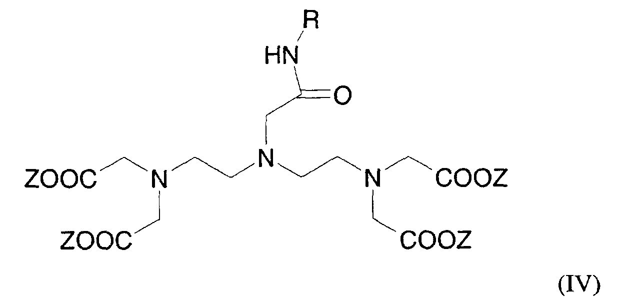 worin Z unabhängig voneinander für ein Wasserstoffatom oder ein Metallionenäquivalent eines Elementes der Ordnungszahlen 57 bis 83 und R für einen offenkettigen oder cyclischen, verzweigten oder unverzweigten C₁-C₁₀-Alkylrest steht, welcher mindestens einen aromatischen Ring sowie gegebenenfalls 1 bis 5 Sauerstoffatome, 1 bis 3 Carboxygruppen und/oder 1 bis 3 Amidgruppen enthält.

9. Verbindungen nach Anspruch 8, **dadurch gekennzeichnet, daß** R für einen der Reste 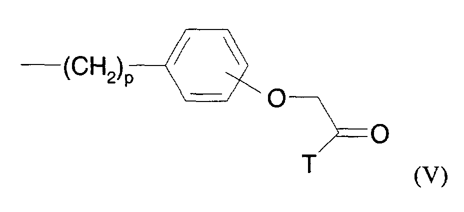 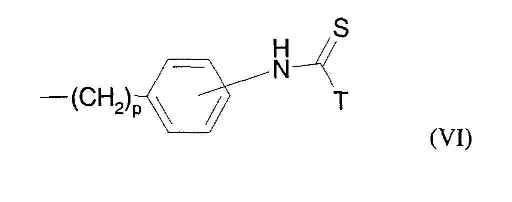 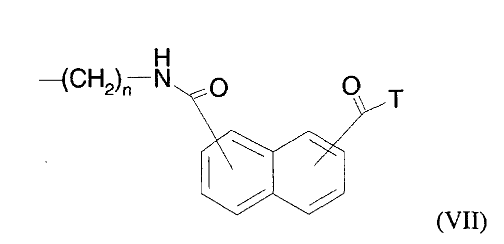 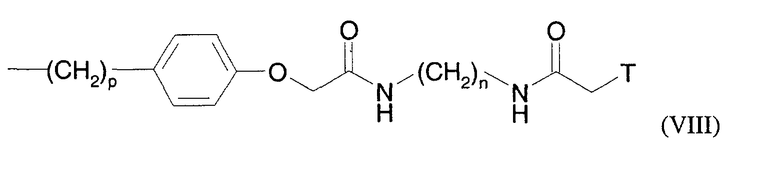 steht, worin T jeweils die Verknüpfungsstelle mit dem Peptid darstellt, p für eine Zahl 0 oder 1 und n für eine Zahl zwischen 2 und 6 steht.

10. Verbindungen nach Anspruch 1, **dadurch gekennzeichnet, daß** die cyclischen Polyaminopolycarbonsäuren Verbindungen der allgemeinen Formel (IX) sind: 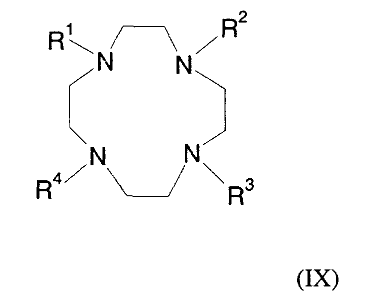 worin R¹ einen Rest ―CHR⁵-COM darstellt, worin M für eine OZ-Gruppe steht, mit Z in der Bedeutung von Anspruch 4, oder die Verknüpfung zum Peptid darstellt, und worin R⁵ für einen Rest (VIII) oder für eine C₁-C₂₀-Alkylgruppe steht, welche mindestens eine Arylgruppe oder einen Heteroaromaten, welche gegebenenfalls mit einem Halogenatom substituiert sein können, und mindestens eine weitere COOZ-Gruppe oder eine Isothiocyanatgruppe enthält, und welche gegebenenfalls 1 bis 3 Sauerstoffatome und/oder 1 bis 3 Amidgruppen enthält,
und worin R² bis R⁴ unabhängig voneinander einen Rest CH₂COOZ, einen Phosphonsäurerest oder eine Gruppe ―(CH₂)ₚ-Y darstellen, in der p für 0 oder 1 steht und Y einen gegebenenfalls substituierten Heteroaromaten darstellt.

11. Verbindungen nach Anspruch 1, **dadurch gekennzeichnet, daß** die cyclischen Polyaminopolycarbonsäuren Verbindungen der allgemeinen Formel (X) sind: 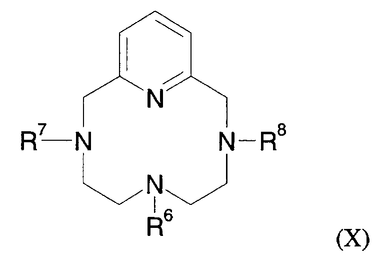 worin R⁶ einen Rest -CHR⁹-COM darstellt, worin M für eine OZ-Gruppe steht, mit Z in der Bedeutung von Anspruch 4, oder die Verknüpfung zum Peptid darstellt,
und worin R⁹ für einen Rest (VIII) oder eine C₁-C₆-Alkylgruppe steht, welche gegebenenfalls eine weitere COOH-Gruppe oder eine Isothiocyanatgruppe enthält, und welche gegebenenfalls 1 bis 2 Sauerstoffatome und/oder 1 bis 2 Amidgruppen enthält,
und worin R⁷ und R⁸ unabhängig voneinander einen Rest CH₂COOZ oder einen Phosphonsäurerest darstellen.

12. Verbindungen nach Anspruch 1, **dadurch gekennzeichnet, daß** (X)ₘ für die Aminosäuresequenz des nativen vasoaktiven intestinalen Peptides entsprechend HSDAVFTDNYTRLRKQMAVKKYLNSILN oder für Fragmente, Teilsequenzen, Derivate oder Analoga des vasoaktiven intestinalen Peptides, bestehend aus 5 bis 30 Aminosäuren, steht.

13. Verbindungen nach Anspruch 1, **dadurch gekennzeichnet, daß** (X)ₘ für die Aminosäuresequenz des Somatostatins entsprechend AGCKNFFWKTFTSC oder für Fragmente, Teilsequenzen, Derivate oder Analoga des Somatostatins, bestehend aus 5 bis 20 Aminosäuren, steht.

14. Verbindungen nach Anspruch 1, **dadurch gekennzeichnet, daß** (X)ₘ für die Aminosäuresequenz des Neurotensins entsprechend Pyroglutaminsäure-LYENKPRRPYIL oder für Fragmente, Teilsequenzen, Derivate oder Analoga des Neurotensins, bestehend aus 5 bis 20 Aminosäuren, steht.

15. Verbindungen nach Anspruch 12, **dadurch gekennzeichnet, daß** als Fragmente, Teilsequenzen, Derivate oder Analoga des vasoaktiven intestinalen Peptides folgenden Aminosäuresequenzen ausgewählt sind:

RLRKQMAVKKYLNSILN RLRKQMAVKKYLNSIL RLRKQMAVKKYLNSI
LRKQMAVKKYLNSILN LRKQMAVKKYLNSIL LRKQMAVKKYLNSI
RKQMAVKKYLNSILN RKQMAVKKYLNSIL RKQMAVKKYLNSI
KQMAVKKYLNSILN KQMAVKKYLNSIL KQMAVKKYLNSI
QMAVKKYLNSILN QMAVKKYLNSIL QMAVKKYLNSI
MAVKKYLNSILN MAVKKYLNSIL MAVKKYLNSI
AVKKYLNSILN AVKKYLNSIL AVKKYLNSI

RLRKQMAVKKYLNS | RLRKQMAVKKYLN | RLRKQMAVKKYL
LRKQMAVKKYLNS | LRKQMAVKKYLN | LRKQMAVKKYL
RKQMAVKKYLNS | RKQMAVKKYLN | RKQMAVKKYL
KQMAVKKYLNS | KQMAVKKYLN | KQMAVKKYL
QMAVKKYLNS | QMAVKKYLN | QMAVKKYL
MAVKKYLNS | MAVKKYLN | MAVKKYL
AVKKYLNS | AVKKYLN | AVKKYL

16. Verbindungen nach mindestens einem der vorhergehenden Ansprüche, **dadurch gekennzeichnet, daß** 1 bis m Aminosäuren unabhängig voneinander gegen ihre jeweilige D-Aminosäure oder gegen andere L- oder D-Aminosäuren ausgetauscht sein können, wobei m die oben angegebene Bedeutung hat.

17. Verbindungen nach mindestens einem der vorhergehenden Ansprüche, **dadurch gekennzeichnet, daß** sämtliche Aminosäuren (X)ₘ gegen ihre jeweilige D-Aminosäure ausgetauscht sind.

18. Verbindungen nach mindestens einem der vorhergehenden Ansprüche, **dadurch gekennzeichnet, daß** als Fragmente, Teilsequenzen, Derivate oder Analoga des vasoaktiven intestinalen Peptides retrosynthetische Aminosäuresequenzen ausgewählt sind.

19. Verbindungen nach mindestens einem der vorhergehenden Ansprüche, **dadurch gekennzeichnet, daß** als Fragmente, Teilsequenzen, Derivate oder Analoga des vasoaktiven intestinalen Peptides retrosynthetische Aminosäuresequenzen, bei denen 1 bis m Aminosäuren gegen die jeweilige D-Aminosäure ausgetauscht sind, ausgewählt sind, wobei m die oben angegebene Bedeutung hat.

20. Verbindungen nach Anspruch 12, **dadurch gekennzeichnet, daß** als Fragmente, Teilsequenzen, Derivate oder Analoga des vasoaktiven intestinalen Peptides folgende Aminosäuresequenzen ausgewählt sind:

rlrkqmavkkylnsiln | rlrkqmavkkylnsil | rlrkqmavkkylnsi
lrkqmavkkylnsiln | lrkqmavkkylnsil | lrkqmavkkylnsi
rkqmavkkylnsiln | rkqmavkkylnsil | rkqmavkkylnsi
kqmavkkylnsiln | kqmavkkylnsil | kqmavkkylnsi
qmavkkylnsiln | qmavkkylnsil | qmavkkylnsi
mavkkylnsiln | mavkkylnsil | mavkkylnsi
avkkylnsiln | avkkylnsil | avkkylnsi

RLRKQMAvKKyLNSILN RLRKQMAvKKyLNSIL RLRKQMAvKKyLNSI
LRKQMAvKKyLNSILN LRKQMAvKKyLNSIL LRKQMAvKKyLNSI
RKQMAvKKyLNSILN RKQMAvKKyLNSIL RKQMAvKKyLNSI
KQMAvKKyLNSILN KQMAvKKyLNSIL KQMAvKKyLNSI
QMAvKKyLNSILN QMAvKKyLNSIL QMAvKKyLNSI
MAvKKyLNSILN MAvKKyLNSIL MAvKKyLNSI
AvKKyLNSILN AvKKyLNSIL AvKKyLNSI

21. Verbindungen nach Anspruch 12, **dadurch gekennzeichnet, daß** als Analoga des VIP Peptide aus der folgenden Gruppe von Sequenzen ausgewählt sind:

FSDAVFTDNY TRLRKQMAVK KYLNSILN
ISDAVFTDNY TRLRKQMAVK KYLNSILN
LSDAVFTDNY TRLRKQMAVK KYLNSILN
HFDAVFTDNY TRLRKQMAVK KYLNSILN
HHDAVFTDNY TRLRKQMAVK KYLNSILN
HIDAVFTDNY TRLRKQMAVK KYLNSILN
HLDAVFTDNY TRLRKQMAVK KYLNSILN
HMDAVFTDNY TRLRKQMAVK KYLNSILN
HQDAVFTDNY TRLRKQMAVK KYLNSILN
HTDAVFTDNY TRLRKQMAVK KYLNSILN
HVDAVFTDNY TRLRKQMAVK KYLNSILN
HWDAVFTDNY TRLRKQMAVK KYLNSILN
HYDAVFTDNY TRLRKQMAVK KYLNSILN
HSAAVFTDNY TRLRKQMAVK KYLNSILN
HSEAVFTDNY TRLRKQMAVK KYLNSILN
HSFAVFTDNY TRLRKQMAVK KYLNSILN
HSHAVFTDNY TRLRKQMAVK KYLNSILN
HSIAVFTDNY TRLRKQMAVK KYLNSILN
HSLAVFTDNY TRLRKQMAVK KYLNSILN
HSMAVFTDNY TRLRKQMAVK KYLNSILN
HSWAVFTDNY TRLRKQMAVK KYLNSILN
HSDFVFTDNY TRLRKQMAVK KYLNSILN
HSDGVFTDNY TRLRKQMAVK KYLNSILN
HSDMVFTDNY TRLRKQMAVK KYLNSILN
HSDQVFTDNY TRLRKQMAVK KYLNSILN
HSDSVFTDNY TRLRKQMAVK KYLNSILN
HSDWVFTDNY TRLRKQMAVK KYLNSILN
HSDYVFTDNY TRLRKQMAVK KYLNSILN
HSDAFFTDNY TRLRKQMAVK KYLNSILN
HSDAIFTDNY TRLRKQMAVK KYLNSILN
HSDALFTDNY TRLRKQMAVK KYLNSILN

HSDAMFTDNY TRLRKQMAVK KYLNSILN
HSDATFTDNY TRLRKQMAVK KYLNSILN
HSDAWFTDNY TRLRKQMAVK KYLNSILN
HSDAYFTDNY TRLRKQMAVK KYLNSILN
HSDAVKTDNY TRLRKQMAVK KYLNSILN
HSDAVFVDNY TRLRKQMAVK KYLNSILN
HSDAVFWDNY TRLRKQMAVK KYLNSILN
HSDAVFTDNW TRLRKQMAVK KYLNSILN
HSDAVFTDNY TRRRKQMAVK KYLNSILN
HSDAVFTDNY TRWRKQMAVK KYLNSILN
HSDAVFTDNY TRLRFQMAVK KYLNSILN
HSDAVFTDNY TRLRLQMAVK KYLNSILN
HSDAVFTDNY TRLRMQMAVK KYLNSILN
HSDAVFTDNY TRLRRQMAVK KYLNSILN
HSDAVFTDNY TRLRKAMAVK KYLNSILN
HSDAVFTDNY TRLRKFMAVK KYLNSILN
HSDAVFTDNY TRLRKIMAVK KYLNSILN
HSDAVFTDNY TRLRKKMAVK KYLNSILN
HSDAVFTDNY TRLRKLMAVK KYLNSILN
HSDAVFTDNY TRLRKMMAVK KYLNSILN
HSDAVFTDNY TRLRKRMAVK KYLNSILN
HSDAVFTDNY TRLRKVMAVK KYLNSILN
HSDAVFTDNY TRLRKWMAVK KYLNSILN
HSDAVFTDNY TRLRKYMAVK KYLNSILN
HSDAVFTDNY TRLRKQFAVK KYLNSILN
HSDAVFTDNY TRLRKQIAVK KYLNSILN
HSDAVFTDNY TRLRKQKAVK KYLNSILN
HSDAVFTDNY TRLRKQLAVK KYLNSILN
HSDAVFTDNY TRLRKQQAVK KYLNSILN
HSDAVFTDNY TRLRKQRAVK KYLNSILN
HSDAVFTDNY TRLRKQWAVK KYLNSILN
HSDAVFTDNY TRLRKQMFVK KYLNSILN
HSDAVFTDNY TRLRKQMIVK KYLNSILN
HSDAVFTDNY TRLRKQMKVK KYLNSILN
HSDAVFTDNY TRLRKQMLVK KYLNSILN
HSDAVFTDNY TRLRKQMMVK KYLNSILN

HSDAVFTDNY TRLRKQMQVK KYLNSILN
HSDAVFTDNY TRLRKQMRVK KYLNSILN
HSDAVFTDNY TRLRKQMVVK KYLNSILN
HSDAVFTDNY TRLRKQMWVK KYLNSILN
HSDAVFTDNY TRLRKQMYVK KYLNSILN
HSDAVFTDNY TRLRKQMAAK KYLNSILN
HSDAVFTDNY TRLRKQMAIK KYLNSILN
HSDAVFTDNY TRLRKQMALK KYLNSILN
HSDAVFTDNY TRLRKQMAVR KYLNSILN
HSDAVFTDNY TRLRKQMAVK RYLNSILN
HSDAVFTDNY TRLRKQMAVK WYLNSILN
HSDAVFTDNY TRLRKQMAVK KFLNSILN
HSDAVFTDNY TRLRKQMAVK KWLNSILN
HSDAVFTDNY TRLRKQMAVK KYLASILN
HSDAVFTDNY TRLRKQMAVK KYLFSILN
HSDAVFTDNY TRLRKQMAVK KYLISILN
HSDAVFTDNY TRLRKQMAVK KYLMSILN
HSDAVFTDNY TRLRKQMAVK KYLSSILN
HSDAVFTDNY TRLRKQMAVK KYLVSILN
HSDAVFTDNY TRLRKQMAVK KYLWSILN
HSDAVFTDNY TRLRKQMAVK KYLNNILN
HSDAVFTDNY TRLRKQMAVK KYLNRILN
HSDAVFTDNY TRLRKQMAVK KYLNWILN
HSDAVFTDNY TRLRKQMAVK KYLNYILN
HSDAVFTDNY TRLRKQMAVK KYLNSLLN
HSDAVFTDNY TRLRKQMAVK KYLNSSLN
HSDAVFTDNY TRLRKQMAVK KYLNSWLN
HSDAVFTDNY TRLRKQMAVK KYLNSYLN
HSDAVFTDNY TRLRKQMAVK KYLNSIFN
HSDAVFTDNY TRLRKQMAVK KYLNSIIN
HSDAVFTDNY TRLRKQMAVK KYLNSIWN
HSDAVFTDNY TRLRKQMAVK KYLNSILW

22. Verbindungen nach Anspruch 12, **dadurch gekennzeichnet, daß** als Analogon des VIP eine Verbindung gemäß folgender Formel ausgewählt ist: HSDAVFTX¹X²Y X³RLRKQMAVK KYLNSILN, worin X¹, X² und X³ jede beliebige Aminosäure darstellen können.

23. Verbindungen nach Anspruch 13, **dadurch gekennzeichnet, daß** als Fragmente, Teilsequenzen, Derivate oder Analoga des Somatostatins folgende Aminosäuresequenzen ausgewählt sind:

AGCKNFFwKTFTSC    AGcKNFFwKTFTSC
AGCKNFFwKTFTSc    AGcKNFFwKTFTSc
CKNFFwKTFTSC    cKNFFwKTFTSC
fFYwKVFT
fCFwKVCT    fcFwKVCT
fCYwKVCT    fcYwKVCT
fCFwKTCT    fcFwKTCT
fCYwKTCT    fcYwKTCT
D-NaI-CYwKVC    D-NaI-cYwKVC
fCywK-Abu-C-NaI    fcywK-Abu-C-NaI

24. Verbindungen nach Anspruch 14, **dadurch gekennzeichnet, daß** als Fragmente, Teilsequenzen, Derivate oder Analoga des Neurotensins folgende Aminosäuresequenzen ausgewählt sind:

pGlu-LYQNKPRRPFIL | pGlu-LYENKPRRPYI
pGlu-LYENKPRRPyIL | pGlu-LYQNKPRRPfIL | pGlu-LYENKPRRPY
pGlu-LYQNKPRRPYIL | pGlu-LYENKPRRPWIL | pGlu-LYENKPRRP
pGlu-LYQNKPRRPyIL | pGlu-LYENKPRRPwIL | pGlu-LYENKPRR
pGlu-LYENKPRRPFIL | pGlu-LYQNKPRRPWIL | pGlu-LYENKPR
pGlu-LYENKPRRPfIL | pGlu-LYQNKPRRPwIL | pGlu-LYENKP

NKPRRPYIL NKPRRPyIL NKPRRPfIL NKPRRPwIL
KPRRPYIL KPRRPyIL KPRRPfIL KPRRPwIL
PRRPYIL PRRPyIL PRRPfIL PRRPwIL
RRPYIL RRPyIL RRPfIL RRPwIL

25. Verbindungen nach einem der vorangegangenen Ansprüche, **dadurch gekennzeichnet, daß** die Verbindungen ein Terbium- oder Europiumkation enthalten.

26. Verwendung der Verbindungen nach mindestens einem der vorangehenden Ansprüche zur In-vivo-Diagnostik von Tumoren, anderen erkrankten Gewebebereichen oder Adenomen mittels optischer Detektionsverfahren, oder zur In-vivo-Fluoreszenzdiagnostik von Tumoren, Tumorzellen und/oder entzündlichen Geweben mittels endoskopischer Verfahren im Gastrointestinaltrakt, Oesophagus, Bronchialtrakt, der Blase oder der Zervix.

27. Verfahren zur endoskopischen In-vivo-Fluoreszenzdiagnostik unter Verwendung der Verbindungen nach Anspruch 1, **dadurch gekennzeichnet, daß** dem Patienten die Verbindungen topisch durch Versprühen im Gastrointestinaltrakt, Oesophagus, der Blase, oder durch Inhalation den Bronchien zugeführt werden,
der nicht gebundene, überschüssige Anteil der Verbindung gegebenenfalls anschließend durch Waschen entfernt wird,
und schließlich die endoskopische Untersuchung durch örtliche Anregung mit einer aus dem Spektralbereich von 250 bis 450 nm ausgewählten Anregungswellenlänge und durch ortsabhängige Detektion der spezifischen, von der Verbindung emittierten Fluoreszenzstrahlung durchgeführt wird.

28. Optisches Diagnostikum zur In-vivo-Diagnostik erkrankter Gewebebereiche, **dadurch gekennzeichnet, daß** es mindestens eine Verbindung nach Anspruch 1 zusammen mit den üblichen Hilfs- und/oder Trägerstoffen sowie Verdünnungsmitteln enthält.

29. Verfahren zur Herstellung von Verbindungen gemäß Anspruch 1, **dadurch gekennzeichnet, daß** zunächst ein Metallkomplex hergestellt wird, welcher durch Aminolyse des entsprechenden Aktivesters an ein Peptid gekoppelt wird.


### Annex to the application documents-subsequently filed sequences listing

SEQUENCE LISTING

<110> BAUER, MICHAEL
      BECKER, ANDREAS
      LICHA, KAI
      BORNHOP, DARRYL
      PLATZEK, JOHANNES

<120> NEW COMPOUNDS FOR FLUORESCENCE DIAGNOSIS

<130> SCH-1755

<140> 09/571,407
<141> 2000-05-15

<160> 241

<170> PatentIn Ver. 2.1

<210> 1
<211> 28
<212> PRT
<213> Unknown Organism

<220>
<223> Description of Unknown Organism: Naturally
      occuring VIP

<400> 1
His Ser Asp Ala Val Phe Thr Asp Asn Tyr Thr Arg Leu Arg Lys Gln
  1               5                  10                  15

Met Ala Val Lys Lys Tyr Leu Asn Ser Ile Leu Asn
             20                  25


<210> 2
<211> 14
<212> PRT
<213> Unknown Organism

<220>
<223> Description of Unknown Organism: Naturally
      occuring somatostatin

<400> 2
Ala Gly Cys Lys Asn Phe Phe Trp Lys Thr Phe Thr Ser Cys
  1               5                  10


<210> 3
<211> 12
<212> PRT
<213> Unknown Organism

<220>
<223> Description of Unknown Organism: Naturally
      occuring neurotensin

<400> 3
Leu Tyr Glu Asn Lys Pro Arg Arg Pro Tyr Ile Leu

1                5                   10


<210> 4
<211> 17
<212> PRT
<213> Artificial Sequence

<220>
<223> Description of Artificial Sequence: Synthetic
      peptide

<400> 4
Arg Leu Arg Lys Gln Met Ala Val Lys Lys Tyr Leu Asn Ser Ile Leu
  1               5                  10                  15

Asn



<210> 5
<211> 16
<212> PRT
<213> Artificial Sequence

<220>
<223> Description of Artificial Sequence: Synthetic
      peptide

<400> 5
Leu Arg Lys Gln Met Ala Val Lys Lys Tyr Leu Asn Ser Ile Leu Asn
  1               5                  10                  15


<210> 6
<211> 15
<212> PRT
<213> Artificial Sequence

<220>
<223> Description of Artificial Sequence: Synthetic
      peptide

<400> 6
Arg Lys Gln Met Ala Val Lys Lys Tyr Leu Asn Ser Ile Leu Asn
  1               5                  10                  15


<210> 7
<211> 14
<212> PRT
<213> Artificial Sequence

<220>
<223> Description of Artificial Sequence: Synthetic
      peptide

<400> 7
Lys Gln Met Ala Val Lys Lys Tyr Leu Asn Ser Ile Leu Asn
  1               5                  10

<210> 8
<211> 13
<212> PRT
<213> Artificial Sequence

<220>
<223> Description of Artificial Sequence: Synthetic
      peptide

<400> 8
Gln Met Ala Val Lys Lys Tyr Leu Asn Ser Ile Leu Asn
  1               5                  10


<210> 9
<211> 12
<212> PRT
<213> Artificial Sequence

<220>
<223> Description of Artificial Sequence: Synthetic
      peptide

<400> 9
Met Ala Val Lys Lys Tyr Leu Asn Ser Ile Leu Asn
  1               5                  10


<210> 10
<211> 11
<212> PRT
<213> Artificial Sequence

<220>
<223> Description of Artificial Sequence: Synthetic
      peptide

<400> 10
Ala Val Lys Lys Tyr Leu Asn Ser Ile Leu Asn
  1               5                  10


<210> 11
<211> 16
<212> PRT
<213> Artificial Sequence

<220>
<223> Description of Artificial Sequence: Synthetic
      peptide

<400> 11
Arg Leu Arg Lys Gln Met Ala Val Lys Lys Tyr Leu Asn Ser Ile Leu
  1               5                  10                  15


<210> 12
<211> 15
<212> PRT
<213> Artificial Sequence

<220>
<223> Description of Artificial Sequence: Synthetic
      peptide

<400> 12
Leu Arg Lys Gln Met Ala Val Lys Lys Tyr Leu Asn Ser Ile Leu
  1               5                  10                  15


<210> 13
<211> 14
<212> PRT
<213> Artificial Sequence

<220>
<223> Description of Artificial Sequence: Synthetic
      peptide

<400> 13
Arg Lys Gln Met Ala Val Lys Lys Tyr Leu Asn Ser Ile Leu
  1               5                  10


<210> 14
<211> 13
<212> PRT
<213> Artificial Sequence

<220>
<223> Description of Artificial Sequence: Synthetic
      peptide

<400> 14
Lys Gln Met Ala Val Lys Lys Tyr Leu Asn Ser Ile Leu
  1               5                  10


<210> 15
<211> 12
<212> PRT
<213> Artificial Sequence

<220>
<223> Description of Artificial Sequence: Synthetic
      peptide

<400> 15
Gln Met Ala Val Lys Lys Tyr Leu Asn Ser Ile Leu
  1               5                  10


<210> 16
<211> 11
<212> PRT
<213> Artificial Sequence

<220>
<223> Description of Artificial Sequence: Synthetic
      peptide

<400> 16
Met Ala Val Lys Lys Tyr Leu Asn Ser Ile Leu
1               5                   10

<210> 17
<211> 10
<212> PRT
<213> Artificial Sequence

<220>
<223> Description of Artificial Sequence: Synthetic
      peptide

<400> 17
Ala Val Lys Lys Tyr Leu Asn Ser Ile Leu
1               5                   10

<210> 18
<211> 15
<212> PRT
<213> Artificial Sequence

<220>
<223> Description of Artificial Sequence: Synthetic
      peptide

<400> 18
Arg Leu Arg Lys Gln Met Ala Val Lys Lys Tyr Leu Asn Ser Ile
1               5                   10                  15

<210> 19
<211> 14
<212> PRT
<213> Artificial Sequence

<220>
<223> Description of Artificial Sequence: Synthetic
      peptide

<400> 19
Leu Arg Lys Gln Met Ala Val Lys Lys Tyr Leu Asn Ser Ile
1               5                   10

<210> 20
<211> 13
<212> PRT
<213> Artificial Sequence

<220>
<223> Description of Artificial Sequence: Synthetic
      peptide

<400> 20
Arg Lys Gln Met Ala Val Lys Lys Tyr Leu Asn Ser Ile
1               5                   10

<210> 21
<211> 12
<212> PRT
<213> Artificial Sequence

<220>
<223> Description of Artificial Sequence: Synthetic
      peptide

<400> 21
Lys Gln Met Ala Val Lys Lys Tyr Leu Asn Ser Ile
  1               5                   10


<210> 22
<211> 11
<212> PRT
<213> Artificial Sequence

<220>
<223> Description of Artificial Sequence: Synthetic
      peptide

<400> 22
Gln Met Ala Val Lys Lys Tyr Leu Asn Ser Ile
  1               5                   10


<210> 23
<211> 10
<212> PRT
<213> Artificial Sequence

<220>
<223> Description of Artificial Sequence: Synthetic
      peptide

<400> 23
Met Ala Val Lys Lys Tyr Leu Asn Ser Ile
  1               5                   10


<210> 24
<211> 9
<212> PRT
<213> Artificial Sequence

<220>
<223> Description of Artificial Sequence: Synthetic
      peptide

<400> 24
Ala Val Lys Lys Tyr Leu Asn Ser Ile
  1               5


<210> 25
<211> 14
<212> PRT
<213> Artificial Sequence

<220>
<223> Description of Artificial Sequence: Synthetic
      peptide

<400> 25
Arg Leu Arg Lys Gln Met Ala Val Lys Lys Tyr Leu Asn Ser
  1               5                   10


<210> 26
<211> 13
<212> PRT
<213> Artificial Sequence

<220>
<223> Description of Artificial Sequence: Synthetic
      peptide

<400> 26
Leu Arg Lys Gln Met Ala Val Lys Lys Tyr Leu Asn Ser
  1               5                   10


<210> 27
<211> 12
<212> PRT
<213> Artificial Sequence

<220>
<223> Description of Artificial Sequence: Synthetic
      peptide

<400> 27
Arg Lys Gln Met Ala Val Lys Lys Tyr Leu Asn Ser
  1               5                   10


<210> 28
<211> 11
<212> PRT
<213> Artificial Sequence

<220>
<223> Description of Artificial Sequence: Synthetic
      peptide

<400> 28
Lys Gln Met Ala Val Lys Lys Tyr Leu Asn Ser
  1               5                   10


<210> 29
<211> 10
<212> PRT
<213> Artificial Sequence

<220>
<223> Description of Artificial Sequence: Synthetic
      peptide

<400> 29

Gln Met Ala Val Lys Lys Tyr Leu Asn Ser
1               5                   10

<210> 30
<211> 9
<212> PRT
<213> Artificial Sequence

<220>
<223> Description of Artificial Sequence: Synthetic
      peptide

<400> 30
Met Ala Val Lys Lys Tyr Leu Asn Ser
1               5

<210> 31
<211> 8
<212> PRT
<213> Artificial Sequence

<220>
<223> Description of Artificial Sequence: Synthetic
      peptide

<400> 31
Ala Val Lys Lys Tyr Leu Asn Ser
1               5

<210> 32
<211> 13
<212> PRT
<213> Artificial Sequence

<220>
<223> Description of Artificial Sequence: Synthetic
      peptide

<400> 32
Arg Leu Arg Lys Gln Met Ala Val Lys Lys Tyr Leu Asn
1               5                   10

<210> 33
<211> 12
<212> PRT
<213> Artificial Sequence

<220>
<223> Description of Artificial Sequence: Synthetic
      peptide

<400> 33
Leu Arg Lys Gln Met Ala Val Lys Lys Tyr Leu Asn
1               5                   10

<210> 34

<211> 11
<212> PRT
<213> Artificial Sequence

<220>
<223> Description of Artificial Sequence: Synthetic
      peptide

<400> 34
Arg Lys Gln Met Ala Val Lys Lys Tyr Leu Asn
  1               5                  10


<210> 35
<211> 10
<212> PRT
<213> Artificial Sequence

<220>
<223> Description of Artificial Sequence: Synthetic
      peptide

<400> 35
Lys Gln Met Ala Val Lys Lys Tyr Leu Asn
  1               5                  10


<210> 36
<211> 9
<212> PRT
<213> Artificial Sequence

<220>
<223> Description of Artificial Sequence: Synthetic
      peptide

<400> 36
Gln Met Ala Val Lys Lys Tyr Leu Asn
  1               5


<210> 37
<211> 8
<212> PRT
<213> Artificial Sequence

<220>
<223> Description of Artificial Sequence: Synthetic
      peptide

<400> 37
Met Ala Val Lys Lys Tyr Leu Asn
  1               5


<210> 38
<211> 7
<212> PRT
<213> Artificial Sequence

<220>

<223> Description of Artificial Sequence: Synthetic
      peptide

<400> 38
Ala Val Lys Lys Tyr Leu Asn
  1               5


<210> 39
<211> 12
<212> PRT
<213> Artificial Sequence

<220>
<223> Description of Artificial Sequence: Synthetic
      peptide

<400> 39
Arg Leu Arg Lys Gln Met Ala Val Lys Lys Tyr Leu
  1               5                  10


<210> 40
<211> 11
<212> PRT
<213> Artificial Sequence

<220>
<223> Description of Artificial Sequence: Synthetic
      peptide

<400> 40
Leu Arg Lys Gln Met Ala Val Lys Lys Tyr Leu
  1               5                  10


<210> 41
<211> 10
<212> PRT
<213> Artificial Sequence

<220>
<223> Description of Artificial Sequence: Synthetic
      peptide

<400> 41
Arg Lys Gln Met Ala Val Lys Lys Tyr Leu
  1               5                  10


<210> 42
<211> 9
<212> PRT
<213> Artificial Sequence

<220>
<223> Description of Artificial Sequence: Synthetic
      peptide

<400> 42
Lys Gln Met Ala Val Lys Lys Tyr Leu

1                 5


<210> 43
<211> 8
<212> PRT
<213> Artificial Sequence

<220>
<223> Description of Artificial Sequence: Synthetic
      peptide

<400> 43
Gln Met Ala Val Lys Lys Tyr Leu
  1               5


<210> 44
<211> 7
<212> PRT
<213> Artificial Sequence

<220>
<223> Description of Artificial Sequence: Synthetic
      peptide

<400> 44
Met Ala Val Lys Lys Tyr Leu
  1               5


<210> 45
<211> 6
<212> PRT
<213> Artificial Sequence

<220>
<223> Description of Artificial Sequence: Synthetic
      peptide

<400> 45
Ala Val Lys Lys Tyr Leu
  1               5


<210> 46
<211> 17
<212> PRT
<213> Artificial Sequence

<220>
<223> Description of Artificial Sequence: Synthetic
      peptide

<400> 46
Arg Leu Arg Lys Gln Met Ala Val Lys Lys Tyr Leu Asn Ser Ile Leu
  1               5                  10                  15

Asn

<210> 47
<211> 16
<212> PRT
<213> Artificial Sequence

<220>
<223> Description of Artificial Sequence: Synthetic
      peptide

<400> 47
Leu Arg Lys Gln Met Ala Val Lys Lys Tyr Leu Asn Ser Ile Leu Asn
1               5                   10                  15


<210> 48
<211> 15
<212> PRT
<213> Artificial Sequence

<220>
<223> Description of Artificial Sequence: Synthetic
      peptide

<400> 48
Arg Lys Gln Met Ala Val Lys Lys Tyr Leu Asn Ser Ile Leu Asn
1               5                   10                  15


<210> 49
<211> 14
<212> PRT
<213> Artificial Sequence

<220>
<223> Description of Artificial Sequence: Synthetic
      peptide

<400> 49
Lys Gln Met Ala Val Lys Lys Tyr Leu Asn Ser Ile Leu Asn
1               5                   10


<210> 50
<211> 13
<212> PRT
<213> Artificial Sequence

<220>
<223> Description of Artificial Sequence: Synthetic
      peptide

<400> 50
Gln Met Ala Val Lys Lys Tyr Leu Asn Ser Ile Leu Asn
1               5                   10


<210> 51
<211> 12
<212> PRT
<213> Artificial Sequence

<220>
<223> Description of Artificial Sequence: Synthetic
      peptide

<400> 51
Met Ala Val Lys Lys Tyr Leu Asn Ser Ile Leu Asn
  1               5                  10


<210> 52
<211> 16
<212> PRT
<213> Artificial Sequence

<220>
<223> Description of Artificial Sequence: Synthetic
      peptide

<400> 52
Arg Leu Arg Lys Gln Met Ala Val Lys Lys Tyr Leu Asn Ser Ile Leu
  1               5                  10                  15


<210> 53
<211> 15
<212> PRT
<213> Artificial Sequence

<220>
<223> Description of Artificial Sequence: Synthetic
      peptide

<400> 53
Leu Arg Lys Gln Met Ala Val Lys Lys Tyr Leu Asn Ser Ile Leu
  1               5                  10                  15


<210> 54
<211> 14
<212> PRT
<213> Artificial Sequence

<220>
<223> Description of Artificial Sequence: Synthetic
      peptide

<400> 54
Arg Lys Gln Met Ala Val Lys Lys Tyr Leu Asn Ser Ile Leu
  1               5                  10


<210> 55
<211> 13
<212> PRT
<213> Artificial Sequence

<220>
<223> Description of Artificial Sequence: Synthetic
      peptide

<400> 55
Lys Gln Met Ala Val Lys Lys Tyr Leu Asn Ser Ile Leu
1               5                   10

<210> 56
<211> 12
<212> PRT
<213> Artificial Sequence

<220>
<223> Description of Artificial Sequence: Synthetic
      peptide

<400> 56
Gln Met Ala Val Lys Lys Tyr Leu Asn Ser Ile Leu
1               5                   10

<210> 57
<211> 11
<212> PRT
<213> Artificial Sequence

<220>
<223> Description of Artificial Sequence: Synthetic
      peptide

<400> 57
Met Ala Val Lys Lys Tyr Leu Asn Ser Ile Leu
1               5                   10

<210> 58
<211> 15
<212> PRT
<213> Artificial Sequence

<220>
<223> Description of Artificial Sequence: Synthetic
      peptide

<400> 58
Arg Leu Arg Lys Gln Met Ala Val Lys Lys Tyr Leu Asn Ser Ile
1               5                   10                  15

<210> 59
<211> 14
<212> PRT
<213> Artificial Sequence

<220>
<223> Description of Artificial Sequence: Synthetic
      peptide

<400> 59
Leu Arg Lys Gln Met Ala Val Lys Lys Tyr Leu Asn Ser Ile
1               5                   10

<210> 60
<211> 13
<212> PRT
<213> Artificial Sequence

<220>
<223> Description of Artificial Sequence: Synthetic
      peptide

<400> 60
Arg Lys Gln Met Ala Val Lys Lys Tyr Leu Asn Ser Ile
  1               5                   10


<210> 61
<211> 12
<212> PRT
<213> Artificial Sequence

<220>
<223> Description of Artificial Sequence: Synthetic
      peptide

<400> 61
Lys Gln Met Ala Val Lys Lys Tyr Leu Asn Ser Ile
  1               5                   10


<210> 62
<211> 11
<212> PRT
<213> Artificial Sequence

<220>
<223> Description of Artificial Sequence: Synthetic
      peptide

<400> 62
Gln Met Ala Val Lys Lys Tyr Leu Asn Ser Ile
  1               5                   10


<210> 63
<211> 10
<212> PRT
<213> Artificial Sequence

<220>
<223> Description of Artificial Sequence: Synthetic
      peptide

<400> 63
Met Ala Val Lys Lys Tyr Leu Asn Ser Ile
  1               5                   10


<210> 64
<211> 11
<212> PRT
<213> Artificial Sequence

<220>
<223> Description of Artificial Sequence: Synthetic
      peptide

<400> 64
Ala Val Lys Lys Tyr Leu Asn Ser Ile Leu Asn
  1               5                   10


<210> 65
<211> 10
<212> PRT
<213> Artificial Sequence

<220>
<223> Description of Artificial Sequence: Synthetic
      peptide

<400> 65
Ala Val Lys Lys Tyr Leu Asn Ser Ile Leu
  1               5                   10


<210> 66
<211> 9
<212> PRT
<213> Artificial Sequence

<220>
<223> Description of Artificial Sequence: Synthetic
      peptide

<400> 66
Ala Val Lys Lys Tyr Leu Asn Ser Ile
  1               5


<210> 67
<211> 17
<212> PRT
<213> Artificial Sequence

<220>
<223> Description of Artificial Sequence: Synthetic
      peptide

<400> 67
Arg Leu Arg Lys Gln Met Ala Val Lys Lys Tyr Leu Asn Ser Ile Leu
  1               5                   10                  15

Asn


<210> 68
<211> 16
<212> PRT
<213> Artificial Sequence

<220>
<223> Description of Artificial Sequence: Synthetic

peptide

<400> 68
Leu Arg Lys Gln Met Ala Val Lys Lys Tyr Leu Asn Ser Ile Leu Asn
1               5                   10                  15

<210> 69
<211> 15
<212> PRT
<213> Artificial Sequence

<220>
<223> Description of Artificial Sequence: Synthetic
      peptide

<400> 69
Arg Lys Gln Met Ala Val Lys Lys Tyr Leu Asn Ser Ile Leu Asn
1               5                   10                  15

<210> 70
<211> 14
<212> PRT
<213> Artificial Sequence

<220>
<223> Description of Artificial Sequence: Synthetic
      peptide

<400> 70
Lys Gln Met Ala Val Lys Lys Tyr Leu Asn Ser Ile Leu Asn
1               5                   10

<210> 71
<211> 13
<212> PRT
<213> Artificial Sequence

<220>
<223> Description of Artificial Sequence: Synthetic
      peptide

<400> 71
Gln Met Ala Val Lys Lys Tyr Leu Asn Ser Ile Leu Asn
1               5                   10

<210> 72
<211> 12
<212> PRT
<213> Artificial Sequence

<220>
<223> Description of Artificial Sequence: Synthetic
      peptide

<400> 72
Met Ala Val Lys Lys Tyr Leu Asn Ser Ile Leu Asn
1               5                   10

<210> 73
<211> 11
<212> PRT
<213> Artificial Sequence

<220>
<223> Description of Artificial Sequence: Synthetic
      peptide

<400> 73
Ala Val Lys Lys Tyr Leu Asn Ser Ile Leu Asn
  1               5                  10


<210> 74
<211> 16
<212> PRT
<213> Artificial Sequence

<220>
<223> Description of Artificial Sequence: Synthetic
      peptide

<400> 74
Arg Leu Arg Lys Gln Met Ala Val Lys Lys Tyr Leu Asn Ser Ile Leu
  1               5                  10                  15


<210> 75
<211> 15
<212> PRT
<213> Artificial Sequence

<220>
<223> Description of Artificial Sequence: Synthetic
      peptide

<400> 75
Leu Arg Lys Gln Met Ala Val Lys Lys Tyr Leu Asn Ser Ile Leu
  1               5                  10                  15


<210> 76
<211> 14
<212> PRT
<213> Artificial Sequence

<220>
<223> Description of Artificial Sequence: Synthetic
      peptide

<400> 76
Arg Lys Gln Met Ala Val Lys Lys Tyr Leu Asn Ser Ile Leu
  1               5                  10


<210> 77
<211> 13
<212> PRT

<213> Artificial Sequence

<220>
<223> Description of Artificial Sequence: Synthetic
      peptide

<400> 77
Lys Gln Met Ala Val Lys Lys Tyr Leu Asn Ser Ile Leu
  1               5                   10


<210> 78
<211> 12
<212> PRT
<213> Artificial Sequence

<220>
<223> Description of Artificial Sequence: Synthetic
      peptide

<400> 78
Gln Met Ala Val Lys Lys Tyr Leu Asn Ser Ile Leu
  1               5                   10


<210> 79
<211> 11
<212> PRT
<213> Artificial Sequence

<220>
<223> Description of Artificial Sequence: Synthetic
      peptide

<400> 79
Met Ala Val Lys Lys Tyr Leu Asn Ser Ile Leu
  1               5                   10


<210> 80
<211> 10
<212> PRT
<213> Artificial Sequence

<220>
<223> Description of Artificial Sequence: Synthetic
      peptide

<400> 80
Ala Val Lys Lys Tyr Leu Asn Ser Ile Leu
  1               5                   10


<210> 81
<211> 15
<212> PRT
<213> Artificial Sequence

<220>
<223> Description of Artificial Sequence: Synthetic
      peptide

<400> 81
Arg Leu Arg Lys Gln Met Ala Val Lys Lys Tyr Leu Asn Ser Ile
1               5                   10                  15


<210> 82
<211> 14
<212> PRT
<213> Artificial Sequence

<220>
<223> Description of Artificial Sequence: Synthetic
      peptide

<400> 82
Leu Arg Lys Gln Met Ala Val Lys Lys Tyr Leu Asn Ser Ile
1               5                   10


<210> 83
<211> 13
<212> PRT
<213> Artificial Sequence

<220>
<223> Description of Artificial Sequence: Synthetic
      peptide

<400> 83
Arg Lys Gln Met Ala Val Lys Lys Tyr Leu Asn Ser Ile
1               5                   10


<210> 84
<211> 12
<212> PRT
<213> Artificial Sequence

<220>
<223> Description of Artificial Sequence: Synthetic
      peptide

<400> 84
Lys Gln Met Ala Val Lys Lys Tyr Leu Asn Ser Ile
1               5                   10


<210> 85
<211> 11
<212> PRT
<213> Artificial Sequence

<220>
<223> Description of Artificial Sequence: Synthetic
      peptide

<400> 85
Gln Met Ala Val Lys Lys Tyr Leu Asn Ser Ile
1               5                   10

<210> 86
<211> 10
<212> PRT
<213> Artificial Sequence

<220>
<223> Description of Artificial Sequence: Synthetic
      peptide

<400> 86
Met Ala Val Lys Lys Tyr Leu Asn Ser Ile
  1               5                  10


<210> 87
<211> 9
<212> PRT
<213> Artificial Sequence

<220>
<223> Description of Artificial Sequence: Synthetic
      peptide

<400> 87
Ala Val Lys Lys Tyr Leu Asn Ser Ile
  1               5


<210> 88
<211> 28
<212> PRT
<213> Artificial Sequence

<220>
<223> Description of Artificial Sequence: Synthetic
      peptide

<400> 88
Phe Ser Asp Ala Val Phe Thr Asp Asn Tyr Thr Arg Leu Arg Lys Gln
  1               5                  10                  15

Met Ala Val Lys Lys Tyr Leu Asn Ser Ile Leu Asn
             20                  25


<210> 89
<211> 28
<212> PRT
<213> Artificial Sequence

<220>
<223> Description of Artificial Sequence: Synthetic
      peptide

<400> 89
Ile Ser Asp Ala Val Phe Thr Asp Asn Tyr Thr Arg Leu Arg Lys Gln
  1               5                  10                  15

Met Ala Val Lys Lys Tyr Leu Asn Ser Ile Leu Asn
             20                  25

<210> 90
<211> 28
<212> PRT
<213> Artificial Sequence

<220>
<223> Description of Artificial Sequence: Synthetic
      peptide

<400> 90
Leu Ser Asp Ala Val Phe Thr Asp Asn Tyr Thr Arg Leu Arg Lys Gln
  1               5                  10                  15

Met Ala Val Lys Lys Tyr Leu Asn Ser Ile Leu Asn
             20                  25


<210> 91
<211> 28
<212> PRT
<213> Artificial Sequence

<220>
<223> Description of Artificial Sequence: Synthetic
      peptide

<400> 91
His Phe Asp Ala Val Phe Thr Asp Asn Tyr Thr Arg Leu Arg Lys Gln
  1               5                  10                  15

Met Ala Val Lys Lys Tyr Leu Asn Ser Ile Leu Asn
             20                  25


<210> 92
<211> 28
<212> PRT
<213> Artificial Sequence

<220>
<223> Description of Artificial Sequence: Synthetic
      peptide

<400> 92
His His Asp Ala Val Phe Thr Asp Asn Tyr Thr Arg Leu Arg Lys Gln
  1               5                  10                  15

Met Ala Val Lys Lys Tyr Leu Asn Ser Ile Leu Asn
             20                  25


<210> 93
<211> 28
<212> PRT
<213> Artificial Sequence

<220>
<223> Description of Artificial Sequence: Synthetic
      peptide

<400> 93
His Ile Asp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 94
<211> 28
<212> PRT
<213> Artificial Sequence

<220>
<223> Description of Artificial Sequence: Synthetic
      peptide

<400> 94
His Leu Asp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 95
<211> 28
<212> PRT
<213> Artificial Sequence

<220>
<223> Description of Artificial Sequence: Synthetic
      peptide

<400> 95
His Met Asp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 96
<211> 28
<212> PRT
<213> Artificial Sequence

<220>
<223> Description of Artificial Sequence: Synthetic
      peptide

<400> 96
His Gln Asp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 97

<211> 28
<212> PRT
<213> Artificial Sequence

<220>
<223> Description of Artificial Sequence: Synthetic
      peptide

<400> 97
His Thr Asp Ala Val Phe Thr Asp Asn Tyr Thr Arg Leu Arg Lys Gln
 1               5                  10                  15

Met Ala Val Lys Lys Tyr Leu Asn Ser Ile Leu Asn
            20                  25


<210> 98
<211> 28
<212> PRT
<213> Artificial Sequence

<220>
<223> Description of Artificial Sequence: Synthetic
      peptide

<400> 98
His Val Asp Ala Val Phe Thr Asp Asn Tyr Thr Arg Leu Arg Lys Gln
 1               5                  10                  15

Met Ala Val Lys Lys Tyr Leu Asn Ser Ile Leu Asn
            20                  25


<210> 99
<211> 28
<212> PRT
<213> Artificial Sequence

<220>
<223> Description of Artificial Sequence: Synthetic
      peptide

<400> 99
His Trp Asp Ala Val Phe Thr Asp Asn Tyr Thr Arg Leu Arg Lys Gln
 1               5                  10                  15

Met Ala Val Lys Lys Tyr Leu Asn Ser Ile Leu Asn
            20                  25


<210> 100
<211> 28
<212> PRT
<213> Artificial Sequence

<220>
<223> Description of Artificial Sequence: Synthetic
      peptide

<400> 100
His Tyr Asp Ala Val Phe Thr Asp Asn Tyr Thr Arg Leu Arg Lys Gln

1               5                   10                  15

Met Ala Val Lys Lys Tyr Leu Asn Ser Ile Leu Asn
            20                  25


<210> 101
<211> 28
<212> PRT
<213> Artificial Sequence

<220>
<223> Description of Artificial Sequence: Synthetic
      peptide

<400> 101
His Ser Ala Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 102
<211> 28
<212> PRT
<213> Artificial Sequence

<220>
<223> Description of Artificial Sequence: Synthetic
      peptide

<400> 102
His Ser Glu Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 103
<211> 28
<212> PRT
<213> Artificial Sequence

<220>
<223> Description of Artificial Sequence: Synthetic
      peptide

<400> 103
His Ser Phe Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 104
<211> 28
<212> PRT
<213> Artificial Sequence

<220>
<223> Description of Artificial Sequence: Synthetic
      peptide

<400> 104
His Ser His Ala Val Phe Thr Asp Asn Tyr Thr Arg Leu Arg Lys Gln
  1               5                  10                  15

Met Ala Val Lys Lys Tyr Leu Asn Ser Ile Leu Asn
             20                  25


<210> 105
<211> 28
<212> PRT
<213> Artificial Sequence

<220>
<223> Description of Artificial Sequence: Synthetic
      peptide

<400> 105
His Ser Ile Ala Val Phe Thr Asp Asn Tyr Thr Arg Leu Arg Lys Gln
  1               5                  10                  15

Met Ala Val Lys Lys Tyr Leu Asn Ser Ile Leu Asn
             20                  25


<210> 106
<211> 28
<212> PRT
<213> Artificial Sequence

<220>
<223> Description of Artificial Sequence: Synthetic
      peptide

<400> 106
His Ser Leu Ala Val Phe Thr Asp Asn Tyr Thr Arg Leu Arg Lys Gln
  1               5                  10                  15

Met Ala Val Lys Lys Tyr Leu Asn Ser Ile Leu Asn
             20                  25


<210> 107
<211> 28
<212> PRT
<213> Artificial Sequence

<220>
<223> Description of Artificial Sequence: Synthetic
      peptide

<400> 107
His Ser Met Ala Val Phe Thr Asp Asn Tyr Thr Arg Leu Arg Lys Gln
  1               5                  10                  15

Met Ala Val Lys Lys Tyr Leu Asn Ser Ile Leu Asn

20                  25

<210> 108
<211> 28
<212> PRT
<213> Artificial Sequence

<220>
<223> Description of Artificial Sequence: Synthetic
      peptide

<400> 108
His Ser Trp Ala Val Phe Thr Asp Asn Tyr Thr Arg Leu Arg Lys Gln
1               5                   10                  15

Met Ala Val Lys Lys Tyr Leu Asn Ser Ile Leu Asn
            20                  25

<210> 109
<211> 28
<212> PRT
<213> Artificial Sequence

<220>
<223> Description of Artificial Sequence: Synthetic
      peptide

<400> 109
His Ser Asp Phe Val Phe Thr Asp Asn Tyr Thr Arg Leu Arg Lys Gln
1               5                   10                  15

Met Ala Val Lys Lys Tyr Leu Asn Ser Ile Leu Asn
            20                  25

<210> 110
<211> 28
<212> PRT
<213> Artificial Sequence

<220>
<223> Description of Artificial Sequence: Synthetic
      peptide

<400> 110
His Ser Asp Gly Val Phe Thr Asp Asn Tyr Thr Arg Leu Arg Lys Gln
1               5                   10                  15

Met Ala Val Lys Lys Tyr Leu Asn Ser Ile Leu Asn
            20                  25

<210> 111
<211> 28
<212> PRT
<213> Artificial Sequence

<220>
<223> Description of Artificial Sequence: Synthetic

peptide

<400> 111
His Ser Asp Met Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 112
<211> 28
<212> PRT
<213> Artificial Sequence

<220>
<223> Description of Artificial Sequence: Synthetic
      peptide

<400> 112
His Ser Asp Gln Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 113
<211> 28
<212> PRT
<213> Artificial Sequence

<220>
<223> Description of Artificial Sequence: Synthetic
      peptide

<400> 113
His Ser Asp Ser Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 114
<211> 28
<212> PRT
<213> Artificial Sequence

<220>
<223> Description of Artificial Sequence: Synthetic
      peptide

<400> 114
His Ser Asp Trp Val Phe Thr Asp Asn Tyr Thr Arg Leu Arg Lys Gln
1               5                   10                  15

Met Ala Val Lys Lys Tyr Leu Asn Ser Ile Leu Asn
            20                  25

<210> 115
<211> 28
<212> PRT
<213> Artificial Sequence

<220>
<223> Description of Artificial Sequence: Synthetic
      peptide

<400> 115
His Ser Asp Tyr Val Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 116
<211> 28
<212> PRT
<213> Artificial Sequence

<220>
<223> Description of Artificial Sequence: Synthetic
      peptide

<400> 116
His Ser Asp Ala Phe Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 117
<211> 28
<212> PRT
<213> Artificial Sequence

<220>
<223> Description of Artificial Sequence: Synthetic
      peptide

<400> 117
His Ser Asp Ala Ile Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 118
<211> 28
<212> PRT
<213> Artificial Sequence

<220>
<223> Description of Artificial Sequence: Synthetic
      peptide

<400> 118

His Ser Asp Ala Leu Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 119
<211> 28
<212> PRT
<213> Artificial Sequence

<220>
<223> Description of Artificial Sequence: Synthetic
      peptide

<400> 119
His Ser Asp Ala Met Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 120
<211> 28
<212> PRT
<213> Artificial Sequence

<220>
<223> Description of Artificial Sequence: Synthetic
      peptide

<400> 120
His Ser Asp Ala Thr Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 121
<211> 28
<212> PRT
<213> Artificial Sequence

<220>
<223> Description of Artificial Sequence: Synthetic
      peptide

<400> 121
His Ser Asp Ala Trp Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 122
<211> 28
<212> PRT

<213> Artificial Sequence

<220>
<223> Description of Artificial Sequence: Synthetic
      peptide

<400> 122
His Ser Asp Ala Tyr Phe Thr Asp Asn Tyr Thr Arg Leu Arg Lys Gln
1               5                   10                  15

Met Ala Val Lys Lys Tyr Leu Asn Ser Ile Leu Asn
            20                  25


<210> 123
<211> 28
<212> PRT
<213> Artificial Sequence

<220>
<223> Description of Artificial Sequence: Synthetic
      peptide

<400> 123
His Ser Asp Ala Val Lys Thr Asp Asn Tyr Thr Arg Leu Arg Lys Gln
1               5                   10                  15

Met Ala Val Lys Lys Tyr Leu Asn Ser Ile Leu Asn
            20                  25


<210> 124
<211> 28
<212> PRT
<213> Artificial Sequence

<220>
<223> Description of Artificial Sequence: Synthetic
      peptide

<400> 124
His Ser Asp Ala Val Phe Val Asp Asn Tyr Thr Arg Leu Arg Lys Gln
1               5                   10                  15

Met Ala Val Lys Lys Tyr Leu Asn Ser Ile Leu Asn
            20                  25


<210> 125
<211> 28
<212> PRT
<213> Artificial Sequence

<220>
<223> Description of Artificial Sequence: Synthetic
      peptide

<400> 125
His Ser Asp Ala Val Phe Trp Asp Asn Tyr Thr Arg Leu Arg Lys Gln
1               5                   10                  15

Met Ala Val Lys Lys Tyr Leu Asn Ser Ile Leu Asn
            20                  25

<210> 126
<211> 28
<212> PRT
<213> Artificial Sequence

<220>
<223> Description of Artificial Sequence: Synthetic
      peptide

<400> 126
His Ser Asp Ala Val Phe Thr Asp Asn Trp Thr Arg Leu Arg Lys Gln
1               5                   10                  15

Met Ala Val Lys Lys Tyr Leu Asn Ser Ile Leu Asn
            20                  25

<210> 127
<211> 28
<212> PRT
<213> Artificial Sequence

<220>
<223> Description of Artificial Sequence: Synthetic
      peptide

<400> 127
His Ser Asp Ala Val Phe Thr Asp Asn Tyr Thr Arg Arg Arg Lys Gln
1               5                   10                  15

Met Ala Val Lys Lys Tyr Leu Asn Ser Ile Leu Asn
            20                  25

<210> 128
<211> 28
<212> PRT
<213> Artificial Sequence

<220>
<223> Description of Artificial Sequence: Synthetic
      peptide

<400> 128
His Ser Asp Ala Val Phe Thr Asp Asn Tyr Thr Arg Trp Arg Lys Gln
1               5                   10                  15

Met Ala Val Lys Lys Tyr Leu Asn Ser Ile Leu Asn
            20                  25

<210> 129
<211> 28
<212> PRT
<213> Artificial Sequence

<220>

<223> Description of Artificial Sequence: Synthetic
      peptide

<400> 129
His Ser Asp Ala Val Phe Thr Asp Asn Tyr Thr Arg Leu Arg Phe Gln
1               5                   10                  15

Met Ala Val Lys Lys Tyr Leu Asn Ser Ile Leu Asn
            20                  25


<210> 130
<211> 28
<212> PRT
<213> Artificial Sequence

<220>
<223> Description of Artificial Sequence: Synthetic
      peptide

<400> 130
His Ser Asp Ala Val Phe Thr Asp Asn Tyr Thr Arg Leu Arg Leu Gln
1               5                   10                  15

Met Ala Val Lys Lys Tyr Leu Asn Ser Ile Leu Asn
            20                  25


<210> 131
<211> 28
<212> PRT
<213> Artificial Sequence

<220>
<223> Description of Artificial Sequence: Synthetic
      peptide

<400> 131
His Ser Asp Ala Val Phe Thr Asp Asn Tyr Thr Arg Leu Arg Met Gln
1               5                   10                  15

Met Ala Val Lys Lys Tyr Leu Asn Ser Ile Leu Asn
            20                  25


<210> 132
<211> 28
<212> PRT
<213> Artificial Sequence

<220>
<223> Description of Artificial Sequence: Synthetic
      peptide

<400> 132
His Ser Asp Ala Val Phe Thr Asp Asn Tyr Thr Arg Leu Arg Arg Gln
1               5                   10                  15

Met Ala Val Lys Lys Tyr Leu Asn Ser Ile Leu Asn
            20                  25

<210> 133
<211> 28
<212> PRT
<213> Artificial Sequence

<220>
<223> Description of Artificial Sequence: Synthetic
      peptide

<400> 133
His Ser Asp Ala Val Phe Thr Asp Asn Tyr Thr Arg Leu Arg Lys Ala
  1               5                  10                  15

Met Ala Val Lys Lys Tyr Leu Asn Ser Ile Leu Asn
             20                  25


<210> 134
<211> 28
<212> PRT
<213> Artificial Sequence

<220>
<223> Description of Artificial Sequence: Synthetic
      peptide

<400> 134
His Ser Asp Ala Val Phe Thr Asp Asn Tyr Thr Arg Leu Arg Lys Phe
  1               5                  10                  15

Met Ala Val Lys Lys Tyr Leu Asn Ser Ile Leu Asn
             20                  25


<210> 135
<211> 28
<212> PRT
<213> Artificial Sequence

<220>
<223> Description of Artificial Sequence: Synthetic
      peptide

<400> 135
His Ser Asp Ala Val Phe Thr Asp Asn Tyr Thr Arg Leu Arg Lys Ile
  1               5                  10                  15

Met Ala Val Lys Lys Tyr Leu Asn Ser Ile Leu Asn
             20                  25


<210> 136
<211> 28
<212> PRT
<213> Artificial Sequence

<220>
<223> Description of Artificial Sequence: Synthetic
      peptide

<400> 136
His Ser Asp Ala Val Phe Thr Asp Asn Tyr Thr Arg Leu Arg Lys Lys
1               5                   10                  15

Met Ala Val Lys Lys Tyr Leu Asn Ser Ile Leu Asn
            20                  25


<210> 137
<211> 28
<212> PRT
<213> Artificial Sequence

<220>
<223> Description of Artificial Sequence: Synthetic
      peptide

<400> 137
His Ser Asp Ala Val Phe Thr Asp Asn Tyr Thr Arg Leu Arg Lys Leu
1               5                   10                  15

Met Ala Val Lys Lys Tyr Leu Asn Ser Ile Leu Asn
            20                  25


<210> 138
<211> 28
<212> PRT
<213> Artificial Sequence

<220>
<223> Description of Artificial Sequence: Synthetic
      peptide

<400> 138
His Ser Asp Ala Val Phe Thr Asp Asn Tyr Thr Arg Leu Arg Lys Met
1               5                   10                  15

Met Ala Val Lys Lys Tyr Leu Asn Ser Ile Leu Asn
            20                  25


<210> 139
<211> 28
<212> PRT
<213> Artificial Sequence

<220>
<223> Description of Artificial Sequence: Synthetic
      peptide

<400> 139
His Ser Asp Ala Val Phe Thr Asp Asn Tyr Thr Arg Leu Arg Lys Arg
1               5                   10                  15

Met Ala Val Lys Lys Tyr Leu Asn Ser Ile Leu Asn
            20                  25


<210> 140
<211> 28

<212> PRT
<213> Artificial Sequence

<220>
<223> Description of Artificial Sequence: Synthetic
      peptide

<400> 140
His Ser Asp Ala Val Phe Thr Asp Asn Tyr Thr Arg Leu Arg Lys Val
1               5                   10                  15

Met Ala Val Lys Lys Tyr Leu Asn Ser Ile Leu Asn
            20                  25


<210> 141
<211> 28
<212> PRT
<213> Artificial Sequence

<220>
<223> Description of Artificial Sequence: Synthetic
      peptide

<400> 141
His Ser Asp Ala Val Phe Thr Asp Asn Tyr Thr Arg Leu Arg Lys Trp
1               5                   10                  15

Met Ala Val Lys Lys Tyr Leu Asn Ser Ile Leu Asn
            20                  25


<210> 142
<211> 28
<212> PRT
<213> Artificial Sequence

<220>
<223> Description of Artificial Sequence: Synthetic
      peptide

<400> 142
His Ser Asp Ala Val Phe Thr Asp Asn Tyr Thr Arg Leu Arg Lys Tyr
1               5                   10                  15

Met Ala Val Lys Lys Tyr Leu Asn Ser Ile Leu Asn
            20                  25


<210> 143
<211> 28
<212> PRT
<213> Artificial Sequence

<220>
<223> Description of Artificial Sequence: Synthetic
      peptide

<400> 143
His Ser Asp Ala Val Phe Thr Asp Asn Tyr Thr Arg Leu Arg Lys Gln
1               5                   10                  15

Phe Ala Val Lys Lys Tyr Leu Asn Ser Ile Leu Asn
            20                  25

<210> 144
<211> 28
<212> PRT
<213> Artificial Sequence

<220>
<223> Description of Artificial Sequence: Synthetic
      peptide

<400> 144
His Ser Asp Ala Val Phe Thr Asp Asn Tyr Thr Arg Leu Arg Lys Gln
  1               5                  10                  15

Ile Ala Val Lys Lys Tyr Leu Asn Ser Ile Leu Asn
            20                  25

<210> 145
<211> 28
<212> PRT
<213> Artificial Sequence

<220>
<223> Description of Artificial Sequence: Synthetic
      peptide

<400> 145
His Ser Asp Ala Val Phe Thr Asp Asn Tyr Thr Arg Leu Arg Lys Gln
  1               5                  10                  15

Lys Ala Val Lys Lys Tyr Leu Asn Ser Ile Leu Asn
            20                  25

<210> 146
<211> 28
<212> PRT
<213> Artificial Sequence

<220>
<223> Description of Artificial Sequence: Synthetic
      peptide

<400> 146
His Ser Asp Ala Val Phe Thr Asp Asn Tyr Thr Arg Leu Arg Lys Gln
  1               5                  10                  15

Leu Ala Val Lys Lys Tyr Leu Asn Ser Ile Leu Asn
            20                  25

<210> 147
<211> 28
<212> PRT
<213> Artificial Sequence

<220>
<223> Description of Artificial Sequence: Synthetic
      peptide

<400> 147
His Ser Asp Ala Val Phe Thr Asp Asn Tyr Thr Arg Leu Arg Lys Gln
1               5                   10                  15

Gln Ala Val Lys Lys Tyr Leu Asn Ser Ile Leu Asn
            20                  25


<210> 148
<211> 28
<212> PRT
<213> Artificial Sequence

<220>
<223> Description of Artificial Sequence: Synthetic
      peptide

<400> 148
His Ser Asp Ala Val Phe Thr Asp Asn Tyr Thr Arg Leu Arg Lys Gln
1               5                   10                  15

Arg Ala Val Lys Lys Tyr Leu Asn Ser Ile Leu Asn
            20                  25


<210> 149
<211> 28
<212> PRT
<213> Artificial Sequence

<220>
<223> Description of Artificial Sequence: Synthetic
      peptide

<400> 149
His Ser Asp Ala Val Phe Thr Asp Asn Tyr Thr Arg Leu Arg Lys Gln
1               5                   10                  15

Trp Ala Val Lys Lys Tyr Leu Asn Ser Ile Leu Asn
            20                  25


<210> 150
<211> 28
<212> PRT
<213> Artificial Sequence

<220>
<223> Description of Artificial Sequence: Synthetic
      peptide

<400> 150
His Ser Asp Ala Val Phe Thr Asp Asn Tyr Thr Arg Leu Arg Lys Gln
1               5                   10                  15

Met Phe Val Lys Lys Tyr Leu Asn Ser Ile Leu Asn
            20                  25

<210> 151
<211> 28
<212> PRT
<213> Artificial Sequence

<220>
<223> Description of Artificial Sequence: Synthetic
      peptide

<400> 151
His Ser Asp Ala Val Phe Thr Asp Asn Tyr Thr Arg Leu Arg Lys Gln
  1               5                  10                  15

Met Ile Val Lys Lys Tyr Leu Asn Ser Ile Leu Asn
             20                  25


<210> 152
<211> 28
<212> PRT
<213> Artificial Sequence

<220>
<223> Description of Artificial Sequence: Synthetic
      peptide

<400> 152
His Ser Asp Ala Val Phe Thr Asp Asn Tyr Thr Arg Leu Arg Lys Gln
  1               5                  10                  15

Met Lys Val Lys Lys Tyr Leu Asn Ser Ile Leu Asn
             20                  25


<210> 153
<211> 28
<212> PRT
<213> Artificial Sequence

<220>
<223> Description of Artificial Sequence: Synthetic
      peptide

<400> 153
His Ser Asp Ala Val Phe Thr Asp Asn Tyr Thr Arg Leu Arg Lys Gln
  1               5                  10                  15

Met Leu Val Lys Lys Tyr Leu Asn Ser Ile Leu Asn
             20                  25


<210> 154
<211> 28
<212> PRT
<213> Artificial Sequence

<220>
<223> Description of Artificial Sequence: Synthetic
      peptide

<400> 154
His Ser Asp Ala Val Phe Thr Asp Asn Tyr Thr Arg Leu Arg Lys Gln
1               5                   10                  15

Met Met Val Lys Lys Tyr Leu Asn Ser Ile Leu Asn
            20                  25


<210> 155
<211> 28
<212> PRT
<213> Artificial Sequence

<220>
<223> Description of Artificial Sequence: Synthetic
      peptide

<400> 155
His Ser Asp Ala Val Phe Thr Asp Asn Tyr Thr Arg Leu Arg Lys Gln
1               5                   10                  15

Met Gln Val Lys Lys Tyr Leu Asn Ser Ile Leu Asn
            20                  25


<210> 156
<211> 28
<212> PRT
<213> Artificial Sequence

<220>
<223> Description of Artificial Sequence: Synthetic
      peptide

<400> 156
His Ser Asp Ala Val Phe Thr Asp Asn Tyr Thr Arg Leu Arg Lys Gln
1               5                   10                  15

Met Arg Val Lys Lys Tyr Leu Asn Ser Ile Leu Asn
            20                  25


<210> 157
<211> 28
<212> PRT
<213> Artificial Sequence

<220>
<223> Description of Artificial Sequence: Synthetic
      peptide

<400> 157
His Ser Asp Ala Val Phe Thr Asp Asn Tyr Thr Arg Leu Arg Lys Gln
1               5                   10                  15

Met Val Val Lys Lys Tyr Leu Asn Ser Ile Leu Asn
            20                  25


<210> 158

<211> 28
<212> PRT
<213> Artificial Sequence

<220>
<223> Description of Artificial Sequence: Synthetic
      peptide

<400> 158
His Ser Asp Ala Val Phe Thr Asp Asn Tyr Thr Arg Leu Arg Lys Gln
  1               5                  10                  15

Met Trp Val Lys Lys Tyr Leu Asn Ser Ile Leu Asn
             20                  25


<210> 159
<211> 28
<212> PRT
<213> Artificial Sequence

<220>
<223> Description of Artificial Sequence: Synthetic
      peptide

<400> 159
His Ser Asp Ala Val Phe Thr Asp Asn Tyr Thr Arg Leu Arg Lys Gln
  1               5                  10                  15

Met Tyr Val Lys Lys Tyr Leu Asn Ser Ile Leu Asn
             20                  25


<210> 160
<211> 28
<212> PRT
<213> Artificial Sequence

<220>
<223> Description of Artificial Sequence: Synthetic
      peptide

<400> 160
His Ser Asp Ala Val Phe Thr Asp Asn Tyr Thr Arg Leu Arg Lys Gln
  1               5                  10                  15

Met Ala Ala Lys Lys Tyr Leu Asn Ser Ile Leu Asn
             20                  25


<210> 161
<211> 28
<212> PRT
<213> Artificial Sequence

<220>
<223> Description of Artificial Sequence: Synthetic
      peptide

<400> 161
His Ser Asp Ala Val Phe Thr Asp Asn Tyr Thr Arg Leu Arg Lys Gln

1               5                   10                  15

Met Ala Ile Lys Lys Tyr Leu Asn Ser Ile Leu Asn
            20                  25


<210> 162
<211> 28
<212> PRT
<213> Artificial Sequence

<220>
<223> Description of Artificial Sequence: Synthetic
      peptide

<400> 162
His Ser Asp Ala Val Phe Thr Asp Asn Tyr Thr Arg Leu Arg Lys Gln
1               5                   10                  15

Met Ala Leu Lys Lys Tyr Leu Asn Ser Ile Leu Asn
            20                  25


<210> 163
<211> 28
<212> PRT
<213> Artificial Sequence

<220>
<223> Description of Artificial Sequence: Synthetic
      peptide

<400> 163
His Ser Asp Ala Val Phe Thr Asp Asn Tyr Thr Arg Leu Arg Lys Gln
1               5                   10                  15

Met Ala Val Arg Lys Tyr Leu Asn Ser Ile Leu Asn
            20                  25


<210> 164
<211> 28
<212> PRT
<213> Artificial Sequence

<220>
<223> Description of Artificial Sequence: Synthetic
      peptide

<400> 164
His Ser Asp Ala Val Phe Thr Asp Asn Tyr Thr Arg Leu Arg Lys Gln
1               5                   10                  15

Met Ala Val Lys Arg Tyr Leu Asn Ser Ile Leu Asn
            20                  25


<210> 165
<211> 28
<212> PRT
<213> Artificial Sequence

<220>
<223> Description of Artificial Sequence: Synthetic
      peptide

<400> 165
His Ser Asp Ala Val Phe Thr Asp Asn Tyr Thr Arg Leu Arg Lys Gln
1               5                   10                  15

Met Ala Val Lys Trp Tyr Leu Asn Ser Ile Leu Asn
            20                  25


<210> 166
<211> 28
<212> PRT
<213> Artificial Sequence

<220>
<223> Description of Artificial Sequence: Synthetic
      peptide

<400> 166
His Ser Asp Ala Val Phe Thr Asp Asn Tyr Thr Arg Leu Arg Lys Gln
1               5                   10                  15

Met Ala Val Lys Lys Phe Leu Asn Ser Ile Leu Asn
            20                  25


<210> 167
<211> 28
<212> PRT
<213> Artificial Sequence

<220>
<223> Description of Artificial Sequence: Synthetic
      peptide

<400> 167
His Ser Asp Ala Val Phe Thr Asp Asn Tyr Thr Arg Leu Arg Lys Gln
1               5                   10                  15

Met Ala Val Lys Lys Trp Leu Asn Ser Ile Leu Asn
            20                  25


<210> 168
<211> 28
<212> PRT
<213> Artificial Sequence

<220>
<223> Description of Artificial Sequence: Synthetic
      peptide

<400> 168
His Ser Asp Ala Val Phe Thr Asp Asn Tyr Thr Arg Leu Arg Lys Gln
1               5                   10                  15

Met Ala Val Lys Lys Tyr Leu Ala Ser Ile Leu Asn

20                  25

<210> 169
<211> 28
<212> PRT
<213> Artificial Sequence

<220>
<223> Description of Artificial Sequence: Synthetic
      peptide

<400> 169
His Ser Asp Ala Val Phe Thr Asp Asn Tyr Thr Arg Leu Arg Lys Gln
  1               5                  10                  15

Met Ala Val Lys Lys Tyr Leu Phe Ser Ile Leu Asn
             20                  25

<210> 170
<211> 28
<212> PRT
<213> Artificial Sequence

<220>
<223> Description of Artificial Sequence: Synthetic
      peptide

<400> 170
His Ser Asp Ala Val Phe Thr Asp Asn Tyr Thr Arg Leu Arg Lys Gln
  1               5                  10                  15

Met Ala Val Lys Lys Tyr Leu Ile Ser Ile Leu Asn
             20                  25

<210> 171
<211> 28
<212> PRT
<213> Artificial Sequence

<220>
<223> Description of Artificial Sequence: Synthetic
      peptide

<400> 171
His Ser Asp Ala Val Phe Thr Asp Asn Tyr Thr Arg Leu Arg Lys Gln
  1               5                  10                  15

Met Ala Val Lys Lys Tyr Leu Met Ser Ile Leu Asn
             20                  25

<210> 172
<211> 28
<212> PRT
<213> Artificial Sequence

<220>
<223> Description of Artificial Sequence: Synthetic

peptide

<400> 172
His Ser Asp Ala Val Phe Thr Asp Asn Tyr Thr Arg Leu Arg Lys Gln
1               5                   10                  15

Met Ala Val Lys Lys Tyr Leu Ser Ser Ile Leu Asn
            20                  25


<210> 173
<211> 28
<212> PRT
<213> Artificial Sequence

<220>
<223> Description of Artificial Sequence: Synthetic
      peptide

<400> 173
His Ser Asp Ala Val Phe Thr Asp Asn Tyr Thr Arg Leu Arg Lys Gln
1               5                   10                  15

Met Ala Val Lys Lys Tyr Leu Val Ser Ile Leu Asn
            20                  25


<210> 174
<211> 28
<212> PRT
<213> Artificial Sequence

<220>
<223> Description of Artificial Sequence: Synthetic
      peptide

<400> 174
His Ser Asp Ala Val Phe Thr Asp Asn Tyr Thr Arg Leu Arg Lys Gln
1               5                   10                  15

Met Ala Val Lys Lys Tyr Leu Trp Ser Ile Leu Asn
            20                  25


<210> 175
<211> 28
<212> PRT
<213> Artificial Sequence

<220>
<223> Description of Artificial Sequence: Synthetic
      peptide

<400> 175
His Ser Asp Ala Val Phe Thr Asp Asn Tyr Thr Arg Leu Arg Lys Gln
1               5                   10                  15

Met Ala Val Lys Lys Tyr Leu Asn Asn Ile Leu Asn
            20                  25

<210> 176
<211> 28
<212> PRT
<213> Artificial Sequence

<220>
<223> Description of Artificial Sequence: Synthetic
      peptide

<400> 176
His Ser Asp Ala Val Phe Thr Asp Asn Tyr Thr Arg Leu Arg Lys Gln
1               5                   10                  15

Met Ala Val Lys Lys Tyr Leu Asn Arg Ile Leu Asn
            20                  25


<210> 177
<211> 28
<212> PRT
<213> Artificial Sequence

<220>
<223> Description of Artificial Sequence: Synthetic
      peptide

<400> 177
His Ser Asp Ala Val Phe Thr Asp Asn Tyr Thr Arg Leu Arg Lys Gln
1               5                   10                  15

Met Ala Val Lys Lys Tyr Leu Asn Trp Ile Leu Asn
            20                  25


<210> 178
<211> 28
<212> PRT
<213> Artificial Sequence

<220>
<223> Description of Artificial Sequence: Synthetic
      peptide

<400> 178
His Ser Asp Ala Val Phe Thr Asp Asn Tyr Thr Arg Leu Arg Lys Gln
1               5                   10                  15

Met Ala Val Lys Lys Tyr Leu Asn Tyr Ile Leu Asn
            20                  25


<210> 179
<211> 28
<212> PRT
<213> Artificial Sequence

<220>
<223> Description of Artificial Sequence: Synthetic
      peptide

<400> 179

His Ser Asp Ala Val Phe Thr Asp Asn Tyr Thr Arg Leu Arg Lys Gln
1               5                   10                  15

Met Ala Val Lys Lys Tyr Leu Asn Ser Leu Leu Asn
            20                  25


<210> 180
<211> 28
<212> PRT
<213> Artificial Sequence

<220>
<223> Description of Artificial Sequence: Synthetic
      peptide

<400> 180
His Ser Asp Ala Val Phe Thr Asp Asn Tyr Thr Arg Leu Arg Lys Gln
1               5                   10                  15

Met Ala Val Lys Lys Tyr Leu Asn Ser Ser Leu Asn
            20                  25


<210> 181
<211> 28
<212> PRT
<213> Artificial Sequence

<220>
<223> Description of Artificial Sequence: Synthetic
      peptide

<400> 181
His Ser Asp Ala Val Phe Thr Asp Asn Tyr Thr Arg Leu Arg Lys Gln
1               5                   10                  15

Met Ala Val Lys Lys Tyr Leu Asn Ser Trp Leu Asn
            20                  25


<210> 182
<211> 28
<212> PRT
<213> Artificial Sequence

<220>
<223> Description of Artificial Sequence: Synthetic
      peptide

<400> 182
His Ser Asp Ala Val Phe Thr Asp Asn Tyr Thr Arg Leu Arg Lys Gln
1               5                   10                  15

Met Ala Val Lys Lys Tyr Leu Asn Ser Tyr Leu Asn
            20                  25


<210> 183
<211> 28
<212> PRT

<213> Artificial Sequence

<220>
<223> Description of Artificial Sequence: Synthetic
      peptide

<400> 183
His Ser Asp Ala Val Phe Thr Asp Asn Tyr Thr Arg Leu Arg Lys Gln
1               5                   10                  15

Met Ala Val Lys Lys Tyr Leu Asn Ser Ile Phe Asn
            20                  25


<210> 184
<211> 28
<212> PRT
<213> Artificial Sequence

<220>
<223> Description of Artificial Sequence: Synthetic
      peptide

<400> 184
His Ser Asp Ala Val Phe Thr Asp Asn Tyr Thr Arg Leu Arg Lys Gln
1               5                   10                  15

Met Ala Val Lys Lys Tyr Leu Asn Ser Ile Ile Asn
            20                  25


<210> 185
<211> 28
<212> PRT
<213> Artificial Sequence

<220>
<223> Description of Artificial Sequence: Synthetic
      peptide

<400> 185
His Ser Asp Ala Val Phe Thr Asp Asn Tyr Thr Arg Leu Arg Lys Gln
1               5                   10                  15

Met Ala Val Lys Lys Tyr Leu Asn Ser Ile Trp Asn
            20                  25


<210> 186
<211> 28
<212> PRT
<213> Artificial Sequence

<220>
<223> Description of Artificial Sequence: Synthetic
      peptide

<400> 186
His Ser Asp Ala Val Phe Thr Asp Asn Tyr Thr Arg Leu Arg Lys Gln
1               5                   10                  15

Met Ala Val Lys Lys Tyr Leu Asn Ser Ile Leu Trp
            20                  25

<210> 187
<211> 28
<212> PRT
<213> Artificial Sequence

<220>
<223> Description of Artificial Sequence: Formula
      sequence

<220>
<221> MOD_RES
<222> (8)
<223> Any amino acid

<220>
<221> MOD_RES
<222> (9)
<223> Any amino acid

<220>
<221> MOD_RES
<222> (11)
<223> Any amino acid

<400> 187
His Ser Asp Ala Val Phe Thr Xaa Xaa Tyr Xaa Arg Leu Arg Lys Gln
  1               5                  10                  15

Met Ala Val Lys Lys Tyr Leu Asn Ser Ile Leu Asn
            20                  25

<210> 188
<211> 14
<212> PRT
<213> Artificial Sequence

<220>
<223> Description of Artificial Sequence: Synthetic
      peptide

<400> 188
Ala Gly Cys Lys Asn Phe Phe Trp Lys Thr Phe Thr Ser Cys
  1               5                  10

<210> 189
<211> 14
<212> PRT
<213> Artificial Sequence

<220>
<223> Description of Artificial Sequence: Synthetic
      peptide

<400> 189
Ala Gly Cys Lys Asn Phe Phe Trp Lys Thr Phe Thr Ser Cys

1               5                   10


<210> 190
<211> 12
<212> PRT
<213> Artificial Sequence

<220>
<223> Description of Artificial Sequence: Synthetic
      peptide

<400> 190
Cys Lys Asn Phe Phe Trp Lys Thr Phe Thr Ser Cys
1               5                   10


<210> 191
<211> 8
<212> PRT
<213> Artificial Sequence

<220>
<223> Description of Artificial Sequence: Synthetic
      peptide

<400> 191
Phe Phe Tyr Trp Lys Val Phe Thr
1               5


<210> 192
<211> 8
<212> PRT
<213> Artificial Sequence

<220>
<223> Description of Artificial Sequence: Synthetic
      peptide

<400> 192
Phe Cys Phe Trp Lys Val Cys Thr
1               5


<210> 193
<211> 8
<212> PRT
<213> Artificial Sequence

<220>
<223> Description of Artificial Sequence: Synthetic
      peptide

<400> 193
Phe Cys Tyr Trp Lys Val Cys Thr
1               5


<210> 194
<211> 8

<212> PRT
<213> Artificial Sequence

<220>
<223> Description of Artificial Sequence: Synthetic
      peptide

<400> 194
Phe Cys Phe Trp Lys Thr Cys Thr
  1               5


<210> 195
<211> 8
<212> PRT
<213> Artificial Sequence

<220>
<223> Description of Artificial Sequence: Synthetic
      peptide

<400> 195
Phe Cys Tyr Trp Lys Thr Cys Thr
  1               5


<210> 196
<211> 6
<212> PRT
<213> Artificial Sequence

<220>
<223> Description of Artificial Sequence: Synthetic
      peptide

<400> 196
Cys Tyr Trp Lys Val Cys
  1               5


<210> 197
<211> 7
<212> PRT
<213> Artificial Sequence

<220>
<223> Description of Artificial Sequence: Synthetic
      peptide

<220>
<221> MOD_RES
<222> (6)
<223> Abu

<400> 197
Phe Cys Tyr Trp Lys Xaa Cys
  1               5


<210> 198
<211> 14

<212> PRT
<213> Artificial Sequence

<220>
<223> Description of Artificial Sequence: Synthetic
      peptide

<400> 198
Ala Gly Cys Lys Asn Phe Phe Trp Lys Thr Phe Thr Ser Cys
  1               5                  10


<210> 199
<211> 14
<212> PRT
<213> Artificial Sequence

<220>
<223> Description of Artificial Sequence: Synthetic
      peptide

<400> 199
Ala Gly Cys Lys Asn Phe Phe Trp Lys Thr Phe Thr Ser Cys
  1               5                  10


<210> 200
<211> 12
<212> PRT
<213> Artificial Sequence

<220>
<223> Description of Artificial Sequence: Synthetic
      peptide

<400> 200
Cys Lys Asn Phe Phe Trp Lys Thr Phe Thr Ser Cys
  1               5                  10


<210> 201
<211> 8
<212> PRT
<213> Artificial Sequence

<220>
<223> Description of Artificial Sequence: Synthetic
      peptide

<400> 201
Phe Cys Phe Trp Lys Val Cys Thr
  1               5


<210> 202
<211> 8
<212> PRT
<213> Artificial Sequence

<220>
<223> Description of Artificial Sequence: Synthetic

peptide

<400> 202
Phe Cys Tyr Trp Lys Val Cys Thr
1               5


<210> 203
<211> 8
<212> PRT
<213> Artificial Sequence

<220>
<223> Description of Artificial Sequence: Synthetic
      peptide

<400> 203
Phe Cys Phe Trp Lys Thr Cys Thr
1               5


<210> 204
<211> 8
<212> PRT
<213> Artificial Sequence

<220>
<223> Description of Artificial Sequence: Synthetic
      peptide

<400> 204
Phe Cys Tyr Trp Lys Thr Cys Thr
1               5


<210> 205
<211> 6
<212> PRT
<213> Artificial Sequence

<220>
<223> Description of Artificial Sequence: Synthetic
      peptide

<400> 205
Cys Tyr Trp Lys Val Cys
1               5


<210> 206
<211> 7
<212> PRT
<213> Artificial Sequence

<220>
<223> Description of Artificial Sequence: Synthetic
      peptide

<220>
<221> MOD_RES
<222> (6)

<223> Abu

<400> 206
Phe Cys Tyr Trp Lys Xaa Cys
1               5


<210> 207
<211> 13
<212> PRT
<213> Artificial Sequence

<220>
<223> Description of Artificial Sequence: Synthetic
      peptide

<400> 207
Glu Leu Tyr Gln Asn Lys Pro Arg Arg Pro Phe Ile Leu
1               5                   10


<210> 208
<211> 13
<212> PRT
<213> Artificial Sequence

<220>
<223> Description of Artificial Sequence: Synthetic
      peptide

<400> 208
Glu Leu Tyr Glu Asn Lys Pro Arg Arg Pro Tyr Ile Leu
1               5                   10


<210> 209
<211> 13
<212> PRT
<213> Artificial Sequence

<220>
<223> Description of Artificial Sequence: Synthetic
      peptide

<400> 209
Glu Leu Tyr Gln Asn Lys Pro Arg Arg Pro Tyr Ile Leu
1               5                   10


<210> 210
<211> 13
<212> PRT
<213> Artificial Sequence

<220>
<223> Description of Artificial Sequence: Synthetic
      peptide

<400> 210
Glu Leu Tyr Gln Asn Lys Pro Arg Arg Pro Tyr Ile Leu
1               5                   10

<210> 211
<211> 13
<212> PRT
<213> Artificial Sequence

<220>
<223> Description of Artificial Sequence: Synthetic
      peptide

<400> 211
Glu Leu Tyr Glu Asn Lys Pro Arg Arg Pro Phe Ile Leu
  1               5                   10


<210> 212
<211> 13
<212> PRT
<213> Artificial Sequence

<220>
<223> Description of Artificial Sequence: Synthetic
      peptide

<400> 212
Glu Leu Tyr Glu Asn Lys Pro Arg Arg Pro Phe Ile Leu
  1               5                   10


<210> 213
<211> 12
<212> PRT
<213> Artificial Sequence

<220>
<223> Description of Artificial Sequence: Synthetic
      peptide

<400> 213
Glu Leu Tyr Glu Asn Lys Pro Arg Arg Pro Tyr Ile
  1               5                   10


<210> 214
<211> 13
<212> PRT
<213> Artificial Sequence

<220>
<223> Description of Artificial Sequence: Synthetic
      peptide

<400> 214
Glu Leu Tyr Gln Asn Lys Pro Arg Arg Pro Phe Ile Leu
  1               5                   10


<210> 215
<211> 13
<212> PRT

<213> Artificial Sequence

<220>
<223> Description of Artificial Sequence: Synthetic
      peptide

<400> 215
Glu Leu Tyr Glu Asn Lys Pro Arg Arg Pro Trp Ile Leu
  1               5                   10


<210> 216
<211> 13
<212> PRT
<213> Artificial Sequence

<220>
<223> Description of Artificial Sequence: Synthetic
      peptide

<400> 216
Glu Leu Tyr Glu Asn Lys Pro Arg Arg Pro Trp Ile Leu
  1               5                   10


<210> 217
<211> 13
<212> PRT
<213> Artificial Sequence

<220>
<223> Description of Artificial Sequence: Synthetic
      peptide

<400> 217
Glu Leu Tyr Gln Asn Lys Pro Arg Arg Pro Trp Ile Leu
  1               5                   10


<210> 218
<211> 13
<212> PRT
<213> Artificial Sequence

<220>
<223> Description of Artificial Sequence: Synthetic
      peptide

<400> 218
Glu Leu Tyr Gln Asn Lys Pro Arg Arg Pro Trp Ile Leu
  1               5                   10


<210> 219
<211> 11
<212> PRT
<213> Artificial Sequence

<220>
<223> Description of Artificial Sequence: Synthetic
      peptide

<400> 219
Glu Leu Tyr Glu Asn Lys Pro Arg Arg Pro Tyr
1               5                   10


<210> 220
<211> 10
<212> PRT
<213> Artificial Sequence

<220>
<223> Description of Artificial Sequence: Synthetic
      peptide

<400> 220
Glu Leu Tyr Glu Asn Lys Pro Arg Arg Pro
1               5                   10


<210> 221
<211> 9
<212> PRT
<213> Artificial Sequence

<220>
<223> Description of Artificial Sequence: Synthetic
      peptide

<400> 221
Glu Leu Tyr Glu Asn Lys Pro Arg Arg
1               5


<210> 222
<211> 8
<212> PRT
<213> Artificial Sequence

<220>
<223> Description of Artificial Sequence: Synthetic
      peptide

<400> 222
Glu Leu Tyr Glu Asn Lys Pro Arg
1               5


<210> 223
<211> 7
<212> PRT
<213> Artificial Sequence

<220>
<223> Description of Artificial Sequence: Synthetic
      peptide

<400> 223
Glu Leu Tyr Glu Asn Lys Pro
1               5

<210> 224
<211> 9
<212> PRT
<213> Artificial Sequence

<220>
<223> Description of Artificial Sequence: Synthetic
      peptide

<400> 224
Asn Lys Pro Arg Arg Pro Tyr Ile Leu
  1               5


<210> 225
<211> 8
<212> PRT
<213> Artificial Sequence

<220>
<223> Description of Artificial Sequence: Synthetic
      peptide

<400> 225
Lys Pro Arg Arg Pro Tyr Ile Leu
  1               5


<210> 226
<211> 9
<212> PRT
<213> Artificial Sequence

<220>
<223> Description of Artificial Sequence: Synthetic
      peptide

<400> 226
Asn Lys Pro Arg Arg Pro Tyr Ile Leu
  1               5


<210> 227
<211> 8
<212> PRT
<213> Artificial Sequence

<220>
<223> Description of Artificial Sequence: Synthetic
      peptide

<400> 227
Lys Pro Arg Arg Pro Tyr Ile Leu
  1               5


<210> 228
<211> 9
<212> PRT
<213> Artificial Sequence

<220>
<223> Description of Artificial Sequence: Synthetic
      peptide

<400> 228
Asn Lys Pro Arg Arg Pro Phe Ile Leu
  1               5


<210> 229
<211> 8
<212> PRT
<213> Artificial Sequence

<220>
<223> Description of Artificial Sequence: Synthetic
      peptide

<400> 229
Lys Pro Arg Arg Pro Phe Ile Leu
  1               5


<210> 230
<211> 9
<212> PRT
<213> Artificial Sequence

<220>
<223> Description of Artificial Sequence: Synthetic
      peptide

<400> 230
Asn Lys Pro Arg Arg Pro Trp Ile Leu
  1               5


<210> 231
<211> 8
<212> PRT
<213> Artificial Sequence

<220>
<223> Description of Artificial Sequence: Synthetic
      peptide

<400> 231
Lys Pro Arg Arg Pro Trp Ile Leu
  1               5


<210> 232
<211> 7
<212> PRT
<213> Artificial Sequence

<220>
<223> Description of Artificial Sequence: Synthetic
      peptide

<400> 232
Pro Arg Arg Pro Tyr Ile Leu
  1               5


<210> 233
<211> 6
<212> PRT
<213> Artificial Sequence

<220>
<223> Description of Artificial Sequence: Synthetic
      peptide

<400> 233
Arg Arg Pro Tyr Ile Leu
  1               5


<210> 234
<211> 7
<212> PRT
<213> Artificial Sequence

<220>
<223> Description of Artificial Sequence: Synthetic
      peptide

<400> 234
Pro Arg Arg Pro Tyr Ile Leu
  1               5


<210> 235
<211> 6
<212> PRT
<213> Artificial Sequence

<220>
<223> Description of Artificial Sequence: Synthetic
      peptide

<400> 235
Arg Arg Pro Tyr Ile Leu
  1               5


<210> 236
<211> 7
<212> PRT
<213> Artificial Sequence

<220>
<223> Description of Artificial Sequence: Synthetic
      peptide

<400> 236
Pro Arg Arg Pro Phe Ile Leu
  1               5

<210> 237
<211> 6
<212> PRT
<213> Artificial Sequence

<220>
<223> Description of Artificial Sequence: Synthetic
      peptide

<400> 237
Arg Arg Pro Phe Ile Leu
  1               5


<210> 238
<211> 7
<212> PRT
<213> Artificial Sequence

<220>
<223> Description of Artificial Sequence: Synthetic
      peptide

<400> 238
Pro Arg Arg Pro Trp Ile Leu
  1               5


<210> 239
<211> 6
<212> PRT
<213> Artificial Sequence

<220>
<223> Description of Artificial Sequence: Synthetic
      peptide

<400> 239
Arg Arg Pro Trp Ile Leu
  1               5


<210> 240
<211> 14
<212> PRT
<213> Artificial Sequence

<220>
<223> Description of Artificial Sequence: Synthetic
      cyclo peptide

<400> 240
Ala Gly Cys Lys Asn Phe Phe Trp Lys Thr Phe Thr Ser Cys
  1               5                  10


<210> 241
<211> 28
<212> PRT
<213> Artificial Sequence

<220>
<223> Description of Artificial Sequence:
      Cysteine-linked peptide conjugate

<400> 241
His Ser Asp Ala Val Phe Tyr Asp Asn Tyr Thr Arg Leu Arg Lys Glu
  1               5                  10                  15

Cys Ala Val Lys Lys Tyr Leu Asn Ser Ile Leu Asn
             20                  25